(19) Europäisches Patentamt / European Patent Office / Office européen des brevets

(11) EP 2 949 336 B1

(12) # EUROPEAN PATENT SPECIFICATION

(45) Date of publication and mention of the grant of the patent:
**05.09.2018 Bulletin 2018/36**

(21) Application number: **14743304.9**

(22) Date of filing: **27.01.2014**

(51) Int Cl.:
***A61K 38/16*** $^{(2006.01)}$ ***A61K 9/06*** $^{(2006.01)}$
***A61K 47/34*** $^{(2017.01)}$ ***A61K 47/40*** $^{(2006.01)}$

(86) International application number:
**PCT/JP2014/051719**

(87) International publication number:
**WO 2014/115882 (31.07.2014 Gazette 2014/31)**

(54) **STABILIZED PROTEIN GEL PREPARATION**

STABILISIERTES PROTEINGELPRÄPARAT

PRÉPARATION PHARMACEUTIQUE SOUS FORME DE GEL DE PROTÉINE STABILISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2013 JP 2013013596**

(43) Date of publication of application:
**02.12.2015 Bulletin 2015/49**

(73) Proprietors:
- **National University Corporation Kumamoto University**
  **Kumamoto-shi, Kumamoto 860-8555 (JP)**
- **Terumo Kabushiki Kaisha**
  **Tokyo 151-0072 (JP)**

(72) Inventors:
- **ARIMA, Hidetoshi**
  **Kumamoto-shi**
  **Kumamoto 860-8555 (JP)**
- **MOTOYAMA, Keiichi**
  **Kumamoto-shi**
  **Kumamoto 860-8555 (JP)**
- **HIGASHI, Taishi**
  **Kumamoto-shi**
  **Kumamoto 860-8555 (JP)**
- **TAJIMA, Anna**
  **Kumamoto-shi**
  **Kumamoto 860-8555 (JP)**
- **OHSHITA, Naoko**
  **Kumamoto-shi**
  **Kumamoto 860-8555 (JP)**
- **KOYAMA, Sawako**
  **Ashigarakami-gun**
  **Kanagawa 259-0151 (JP)**
- **IIBUCHI, Ruriko**
  **Ashigarakami-gun**
  **Kanagawa 259-0151 (JP)**
- **MIEDA, Shuuhei**
  **Ashigarakami-gun**
  **Kanagawa 259-0151 (JP)**
- **HANDA, Kenji**
  **Ashigarakami-gun**
  **Kanagawa 259-0151 (JP)**

(74) Representative: **Casalonga**
**Casalonga & Partners**
**Bayerstraße 71/73**
**80335 München (DE)**

(56) References cited:
**JP-A- H09 216 810** **JP-A- 2002 146 100**
**JP-A- 2006 506 335** **JP-A- 2007 538 048**
**US-A1- 2002 019 369** **US-A1- 2004 072 799**
**US-A1- 2007 248 674**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field

The present invention relates to a stabilized protein gel preparation and a method of stabilizing a protein.

Background Art

By virtue of the development of gene recombination technology, a variety of active proteins have come to be provided as preparations. Particularly, owing to the evolution of antibody engineering, chimeric antibodies and humanized antibodies have been put into practical use, to produce remarkable therapeutic effects as medicines. Among others, development of antibody preparations has been progressing vigorously. Protein medicines have heretofore been administered generally by intravenous injection. In recent years, however, an increasing number of protein drugs have been provided as subcutaneous injections that can be self-administered, from the viewpoint of convenience for the patient. Such a drug for subcutaneous injection is administered in a smaller dose as compared with intravenous injections, and is therefore indispensably required to contain the protein in an enhanced concentration. Recently, furthermore, from economical and practical points of view, many attempts have been made to reduce the number of times of administration by increasing the protein concentration. Commercialized preparations include those in which the protein concentration is not less than 100 mg/mL.

In these protein preparations with raised protein concentrations, however, the chance of exposure of the protein to a gas-liquid interface and the chance of interactions between protein molecules are increased; especially, there is a high possibility of the occurrence of aggregates during long-time storage or transportation. The occurrence of aggregates would possibly reduce the amount of active protein molecules, change the pharmacokinetics and, further, bring about undesired immunoreactions such as production of anti-drug antibodies after administration (Schellekens, H. Clin. Therapeutics, 24(11) : 1720-1740. 2002) . In view of this, the FDA of the United States and the EMEA of Europe have set guidelines for evaluation of immunogenicity and have called for countermeasures against this problem. In addition, even among low-concentration protein preparations of peptides, hormones and the like, there exist those which are susceptible to aggregation, depending on the kind of protein or the type of preparation; therefore, such protein preparations should be treated with care such as, for example, to avoid severe shaking at the time of dissolution or administration of the preparation or to pass the preparation through a filter.

From the background as above, there is a need, in relation to protein preparations which have high concentrations or protein preparations which are susceptible to aggregation, for enabling reduction of aggregation and a stable preservation over a long period of time.

The problems such as aggregation, denaturation, decomposition, generation of insoluble foreign matter, or lowering in activity are caused in protein preparations by chemical and physical stresses such as variations in pH, variations in temperature, oxidation, agitation, shear, etc. In order to obtain stability of a protein preparation during preservation and transportation thereof, endeavors have been made to render solvent conditions suitable for the protein. As a general method for inhibiting aggregation of proteins, setting of an appropriate pH and addition of various stabilizers have widely been conducted, the additive used as a stabilizer being salts, polyols, surfactants, amino acids, etc. For instance, U.S. Patent No. 4,783,441 and PCT Patent Publication No. WO 2002/013859 disclose a stabilizing effect of surfactants on proteins. Besides, U.S. Patent No. 7,648,702 and Japanese Patent No. 4219932 (U.S. Patent No. 8,496,930) show protein-stabilizing effects of arginine and glycine.

These additives are soluble and should be used only at such levels as not to be toxic to humans. Taking also the compatibility of these additives to the proteins present as active components into consideration, the kind and amount of the additives to be added are naturally limited. Besides, tremendous endeavor and time are needed for finding an appropriate composition on a protein basis. From such a background, the method for stabilization is desirably a stabilizing method that is widely applicable to protein preparations.

As means for enabling long-time stable preservation of proteins, there have also been adopted, for example, a method in which a protein is frozen at an extremely low temperature (-80°C) and preserved and a method in which a protein is preserved in a freeze-dried form. Especially, in the case where sufficient stability of a protein in aqueous solutions cannot be obtained, the freeze drying is the most versatile, practical alternative technique. These methods, however, need reconstitution of the protein into an administration form immediately before use. Therefore, these methods are conspicuously disadvantageous in regard to the patient's convenience, and the freezing and thawing step and/or the dissolving operation may cause denaturation of the protein.

In recent years, on the other hand, there have been attempted stabilizing methods for high-concentration proteins which are based on crystallization or on administration in an emulsion form by preparation of a water-insoluble vehicle. For instance, PCT Patent Publication No. WO 2008/076819 discloses means for stabilizing a human growth hormone by crystallization. In addition, PCT Patent Publication No. WO 99/55310 (U.S. Patent No. 6541606) describes that a stabilizing effect and a drug release controlling effect can be obtained by encapsulating a crystallized protein in a polymeric carrier.

Hydrogel systems for the delivery and controlled release of drugs are described in US 2002/019369 and US 2007/248674. The first one is a system based on cyclodextrine and poly(ethylene glycol) or derivative thereof, whereas the second one is based on polymers of polyoxypropylene and polyoxyethylene.

Summary of Invention

However, even with the technologies described in PCT Patent Publication Nos. WO 2008/076819 and WO 99/55310 (U.S. Patent No. 6541606), it has not been achieved to sufficiently stabilize a protein against a physical or chemical stress.

In view of the aforementioned circumstances, it is an object of the present invention to provide a protein preparation and a method of stabilizing a protein by which a protein can be stabilized against a physical or chemical stress.

Another object of the present invention is to provide a protein preparation and a method of stabilizing a protein by which a protein can be stabilized against a physical or chemical stress for a long period of time.

The present inventors made extensive and intensive researches for solving the above-mentioned problems. As a result, they found out that the above problems can be solved by a gel preparation which includes a protein (contains a protein in an included state) and which is thixotropic. Based on the finding, they have completed the present invention.

Specifically, the objects of the present invention can be achieved by a gel preparation which is thixotropic and in which an included protein is protected against a physical or chemical stress, is inhibited from aggregation, and is thereby let exist stably.

Brief Description of Drawings

[FIG. 1A] FIG. 1A is a $^1$H-NMR spectrum of a gel preparation 2-12 obtained in (2) of Example 2.

[FIG. 1B] FIG. 1B is a $^1$H-NMR spectrum of a gel preparation 2-37 obtained in (2) of Example 2.

[FIG. 2A] FIG. 2A is a diagram representing powder X-ray diffraction patterns of (a) cyclodextrin alone, (b) polyethylene glycol alone, (c) human IgG alone, (d) a polyethylene glycol/cyclodextrin mixture, (e) a polyethylene glycol/cyclodextrin/human IgG mixture, (f) a human IgG-noncontaining gel preparation, and (g) a solid sample 2-12a of a gel preparation 2-12 (human IgG-containing gel preparation) obtained in (1) of Example 2.

[FIG. 2B] FIG. 2B is a diagram representing powder X-ray diffraction patterns of (a) cyclodextrin alone, (b) polyethylene glycol alone, (c) human IgG alone, (d) a polyethylene glycol/cyclodextrin mixture, (e) a polyethylene glycol/cyclodextrin/human IgG mixture, (f) a human IgG-noncontaining gel preparation, and (g) a solid sample 2-37a of a gel preparation 2-37 (human IgG-containing gel preparation) obtained in (1) of Example 2.

[FIG. 3A] FIG. 3A is a diagram showing a peak value in powder X-ray diffraction and the result of assignment of indices, of the solid sample 2-12a of the gel preparation 2-12 obtained in Example 2.

[FIG. 3B] FIG. 3B is a diagram showing a peak value in powder X-ray diffraction and the result of assignment of indices, of the solid sample 2-37a of the gel preparation 2-37 obtained in Example 2.

[FIG. 4A] FIG. 4A is a diagram representing respective FT-IR spectra of (a) human IgG alone, (b) cyclodextrin alone, and (c) the solid sample 2-12a of the gel preparation 2-12 (human IgG-containing gel preparation) obtained in (1) of Example 2.

[FIG. 4B] FIG. 4B is a diagram representing respective FT-IR spectra of (a) human IgG alone, (b) cyclodextrin alone, and (c) the solid sample 2-37a of the gel preparation 2-37 (human IgG-containing gel preparation) obtained in (1) of Example 2.

[FIG. 5A] FIG. 5A is a diagram representing a variation generated in the viscosity of a gel 12 of Example 3 upon exertion of a stress on the gel at a share rate (100/s).

[FIG. 5B] FIG. 5B is a diagram representing a variation generated in the viscosity of a gel 37 of Example 3 upon exertion of a stress on the gel at a share rate (100/s).

[FIG. 6A] FIG. 6A is a diagram representing variations in the viscosity of a gel after exertion of a stress on a gel preparation 4-12 obtained in Example 4.

[FIG. 6B] FIG. 6B is a diagram representing variations in the viscosity of a gel after exertion of a stress on a gel preparation 4-37 obtained in Example 4.

[FIG. 7] FIG. 7 is a diagram representing inhibitory effects on aggregation which would be induced by an agitation stress, of a human IgG-containing gel preparation 8-12 obtained in Example 8, a preparation obtained by excluding PEG20000 therefrom ($\alpha$-CyD + IgG), a human IgG-containing gel preparation 8-37 obtained in Example 8, a preparation obtained by excluding PEG20000 therefrom ($\gamma$-CyD + IgG), a preparation obtained by excluding cyclodextrin therefrom (PEG + IgG), and a preparation obtained by excluding cyclodextrin and PEG 20000 therefrom (IgG solution).

[FIG. 8] FIG. 8 is a diagram representing inhibitory effects on aggregation which would be induced by an agitation stress, of gel preparations of formulations 2, 4, 5, 6, 11, 12, 18, 26, 33, and 37 containing the human IgG in Example 9.

[FIG. 9] FIG. 9 is a diagram representing inhibitory effects on aggregation which would be induced by an agitation stress, of gel preparations of formulations 2, 4, 5, 6, 11, 12, 16, 18, 25, 26, 33, and 37 containing the human IgG in Example 10.

[FIG. 10] FIG. 10 is a diagram representing inhibitory effects on aggregation which would be induced by a thermal stress, of gel preparations of formulations 12 and 37 containing the human IgG in Example 11.

[FIG. 11] FIG. 11 is a diagram representing an inhibitory effect on aggregation which would be induced by an agitation stress, of a gel preparation of formulation 42 containing the human IgG in Example 13.

[FIG. 12] FIG. 12 is a diagram representing an inhibitory effect on aggregation which would be induced by an agitation stress, of a gel preparation of formulation 52 containing the human IgG in Example 14.

[FIG. 13A] FIG. 13A is a diagram representing inhibitory effects on aggregation which would be induced by an agitation stress, of gel preparations of formulations 40 and 50 containing a TNF$\alpha$/LT$\alpha$ receptor fusion protein in Example 24.

[FIG. 13B] FIG. 13B is a diagram representing inhibitory effects on aggregation which would be induced by an agitation stress, of gel preparations of formulations 40 and 50 containing a TNF$\alpha$/LT$\alpha$ receptor fusion protein in Example 24.

[FIG. 14] FIG. 14 is a diagram representing inhibitory effects on denaturation which would be induced by an agitation stress, of gel preparations of formulations 49 and 59 containing an anti-epidermal growth factor receptor (EGFR) antibody in Example 32.

[FIG. 15A] FIG. 15A is a diagram representing powder X-ray diffraction patterns, of (a) $\alpha$-cyclodextrin alone, (b) Poloxamer 338 alone, (c) human IgG alone, (d) a poloxamer 338/$\alpha$-cyclodextrin/human IgG mixture, and (e) a solid sample 34-97a of gel preparation 34-97 (human IgG-containing gel preparation) obtained in Example 34.

[FIG. 15B] FIG. 15B is a diagram representing powder X-ray diffraction patterns, of (f) $\alpha$-cyclodextrin alone, (g) poloxamer 407 alone, (h) human IgG alone, (i) a poloxamer 407/$\alpha$-cyclodextrin/human IgG mixture, and (j) a solid sample 34-98a of gel preparation 34-98 (human IgG-containing gel preparation) obtained in Example 34.

[FIG. 15C] FIG. 15C is a diagram representing powder X-ray diffraction patterns, of (a) $\gamma$-cyclodextrin alone, (b) poloxamer 338 alone, (c) human IgG alone, (d) a poloxamer 338/$\gamma$-cyclodextrin/human IgG mixture, and (e) a solid sample 34-102a of gel preparation 34-102 (human IgG-containing gel preparation) obtained in Example 34.

[FIG. 15D] FIG. 15D is a diagram representing powder X-ray diffraction patterns, of (f) $\gamma$-cyclodextrin alone, (g) poloxamer 407 alone, (h) human IgG alone, (i) a poloxamer 407/$\gamma$-cyclodextrin/human IgG mixture, and (j) a solid sample 34-103a of gel preparation 34-103 (human IgG-containing gel preparation) obtained in Example 34.

[FIG. 16] FIG. 16 is a diagram representing inhibitory effects on aggregation which would be induced by a thermal stress, of gel preparations 35-97 and 35-102 of formulations 97 and 102 containing the human IgG in Example 35.

Mode for Carrying Out the Invention

The present invention relates to a gel preparation according to claim 1 which is thixotropic and in which an included protein is protected against a physical or chemical stress, is inhibited from aggregation, and is thereby let exist stably. Specifically, the present invention relates to a gel preparation containing a protein and a gel form matter which includes the protein so as to protect the protein from a physical or chemical stress, inhibit the protein from aggregating and thereby let the protein exist stably. The gel preparation of the present invention constituted as above-mentioned is widely applicable to various proteins, and is able to stabilize the proteins.

The present invention relates also to a method of stabilizing a protein according to claim 1, the method having including the protein inside a gel form matter which contains one or more kinds of cyclic molecules, wherein the cyclic molecule is cyclodextrin or a derivative thereof, and one or more kinds of linear molecules, wherein the linear molecule is polyethylene glycol.

According to the gel preparation and the stabilizing method of the present invention, there is provided a stable protein-containing composition which can be used in place of, or in combination with, existing techniques such as addition of a stabilizer. The gel preparation of the present invention, especially, reduces aggregation of a protein which would take place in response to thermal and agitation stresses.

Note that herein "thixotropy" means a property of being gradually lowered in viscosity when continuing to receive a shear stress and being gradually raised in viscosity when left to stand still. Therefore, when a matter is said to "be thixotropic" herein, it suffices for the matter to have a property of being lowered in viscosity and being raised in viscosity when receiving a shear stress and when thereafter put in a standing-still state, respectively. Specifically, it is preferable that the matter said to be thixotropic is lowered in viscosity by not less than 10% when receiving a stress and is raised in viscosity by not less than 10% when thereafter put in a standing-still state.

Herein the "physical stress" means a stress induced by agitation, heat or the like, and the expression of "protect against a physical stress" means an inhibitory effect on aggregation of the included protein under stressed conditions due to agitation, heat or the like. More specifically, the expression of "protect against a physical stress" herein means that the remaining rate (average) of the protein after application of a physical stress thereto is at least 10 points higher than that of a control. Note that the control herein refers to a protein solution or to a protein plus gel component (cyclic molecule and/or linear molecule). Here, the remaining rate of the protein is preferably not less than 65%, more preferably not less than 70%, and further preferably not less than 80%.

Furthermore, the "chemical stress" in the present invention means a stress due to pH, oxidation, a metal-catalyzed reaction or the like, and the expression of "protect against a chemical stress" means an inhibitory effect on denaturation of the included protein under stressed conditions due to pH, oxidation, a metal-catalyzed reaction or the like and an inhibitory effect on aggregation of the protein resulting therefrom. Herein, the expression of "protect against a chemical stress" means that the remaining rate of the protein after application of a chemical stress thereto is at least 10 points higher than that of a control. Here, the remaining rate of the protein is preferably not less than 65%, more preferably not less than 70%, and further preferably not less than 80%.

As one aspect of the present embodiment, the gel preparation contains a protein, a diluent, one or more kinds of cyclic molecules, wherein the cyclic molecule is cyclodextrin or a derivative thereof, and one or more kinds of linear molecules, wherein the linear molecule is polyethylene glycol.

Specifically, the present invention provides a new method for stabilizing a protein on the basis of a composite structure of a hydrogel (gel form matter) and the protein.

As aforementioned, the gel preparation in the present embodiment has the gel form matter (for example, a hydrogel composed of the cyclic molecule or molecules, wherein the cyclic molecule is cyclodextrin or a derivative thereof, and one or more polyethylene glycols) physically surrounding the protein to form a stable shape, whereby the chance of mutual contact between the protein molecules or the chance of exposure of the protein molecules to a gas-liquid interface are decreased, and, consequently, undesirable changes such as aggregation, denaturation, decomposition, etc. of the protein can be prevented from occurring during long-time preservation or transportation.

The mechanism of stabilization of the protein by the gel preparation in the present embodiment is considered as follows. Note that the present invention is not to be restricted to or by the following presumption. The gel form matter in the gel preparation in the present embodiment has a plurality of the cyclic molecules penetrated by (threaded on) the linear molecule or molecules, and the cyclic molecules penetrated by a plurality of different linear molecules, which are assembled due to functional groups present on the cyclic molecules, thereby forming a gel as a whole. The functional group is hydroxyl group, in the case where the cyclic molecule is cyclodextrin, and the hydroxyl groups are mutually linked by hydrogen bonds. The plurality of linear molecules form a three-dimensional network through the linkage between the cyclic molecules, and the protein is present in spaces partitioned by the network. As a result of this, it is considered that movements of the protein are restrained, and the chance of mutual contact between the protein molecules or the chance of exposure of the protein molecules to a gas-liquid interface are reduced, whereby undesirable changes such as aggregation, denaturation, decomposition, etc. of the protein can be prevented from occurring during long-time preservation or transportation.

In addition, since the gel form matter in this embodiment is decreased in viscosity when a stress is exerted thereon, the gel form matter can be delivered from in a device to a target place by applying a pressure, without needing any complicated operation. For example, the gel preparation filled in a cylinder of a syringe can be directly administered subcutaneously as an injection.

The protein which can be included (be contained in an included state) in the present invention is not specifically limited, and preferable examples of the protein include those medicines in which stabilization of protein is demanded and materials for those medicines. In other words, the protein is preferably a protein for medical use. By the method of the present invention, a protein preparation can be stabilized. Also, a protein preparation can be prepared by using a protein material stabilized by the method of the present invention.

Besides, heretofore, among proteins, particularly among protein preparations having high concentrations or protein preparations susceptible to aggregation, there has not been a protein preparation which is satisfactory from the viewpoint of enabling reduction of aggregation and a stable preservation for a long period of time. According to the present invention, however, it is possible to provide a protein preparation which enables reduction of aggregation and a stable long-time preservation, in a protein preparation having a high concentration or a protein preparation susceptible to aggregation.

Gel preparations according to the present invention will be described in detail below, but the invention is not restricted to them. Note that while the gel preparation of the present invention includes a protein, a composition which composes a cyclic molecule, wherein the cyclic molecule is cyclodextrin or a derivative thereof, and a polyethylene glycol, buffer components and the like but does not contain a protein will be referred also to simply as "gel."

[Gel preparation]

As one embodiment of the gel preparation of the present invention, the gel preparation contains a protein, a diluent, one or more kinds of cyclic molecules, wherein the cyclic molecule is cyclodextrin or a derivative thereof, and one or more polyethylene glycols.

Besides, in the present invention, the gel preparation preferably has a gel concentration represented by the following formula [1]:

[Mathematical formula 1]

$$\text{Gel concentration } (wt/vol\%) = \frac{Solids\ weight\ of\ gel\ (g)}{Volume\ of\ gel\ (mL)} \times 100 \qquad [1]$$

Solids weight of gel (g): Weight of precipitate (solids) obtained by centrifugation of gel (g)

(the concentration of a gel having the same formulation as the gel preparation except for not containing the protein) of not less than 4 wt/vol% (hereinafter the gel concentration will be also presented simply in the unit [%]). Note that in the above formula [1], the solids weight (g) of gel means the dry weight (g) of a precipitate (solids) obtained by centrifugation of the gel. The gel concentration (%) means the mass (g) of the cyclodextrin molecules or derivatives thereof, and the polyethylene glycols forming the gel, per 100 mL of the gel preparation. Herein the solids weight of gel means the dry weight (g) of the precipitate (solids) obtained by centrifugation of the gel containing the cyclodextrin molecules or derivatives thereof, the polyethylene glycols, and the diluent, and specifically is determined by the following method. A predetermined amount of the cyclic molecules and a predetermined amount of the linear molecules are dispersed in 0.45 mL of a predetermined buffer in a microtube at 25°C, to prepare a gel. The gel is centrifuged (4°C) at 14,000 × G for 45 minutes, to separate the gel into gel form solids and a supernatant, then the separated supernatant is removed, and the solids are dried. The weight (g) of the dried matter is measured, and the weight is made to be the solids weight of the preparation. Note that as an example of the predetermined buffer, there may be mentioned a phosphate buffer (pH: 6.3) prepared by dissolving 0.50 g of disodium hydrogen phosphate, 1.43 g of sodium dihydrogen phosphate dihydrate, 2.92 g of NaCl, 2.63 g of L-arginine hydrochloride, 5.0 g of sucrose in 0.5 L of distilled water.

When the gel concentration of the gel preparation is not less than 4%, the effects of including the protein and inhibiting the aggregates are exhibited sufficiently, so that such a gel concentration is preferable. More preferably, the gel concentration is 7% to 30%.

The gel preparation according to the present embodiment is a preparation which has a yield value, the viscosity of which is lowered with time by application of a stress thereto, and which is thixotropic. Note that herein a force required for causing the gel to flow, measured by use of a rotational viscometer and a cone/plate spindle with a diameter of 2.5 cm (produced by Brookfield Engineering Laboratories, Inc.), is made to be a yield value of the gel in which the protein is not included. The yield value of the gel in which the protein is not included is preferably not more than 5,000 Pa. The yield value of the gel in which the protein is not included is more preferable as it is lower. Therefore, a lower limit for the yield value of the gel in which the protein is not included is not specifically restricted. It suffices for the yield value to be not less than 50 Pa.

[Cyclic Molecule]

The cyclic molecule contained in the gel preparation of the present invention is cyclodextrin or a derivative thereof.

Cyclodextrin (herein referred also to simply as "CyD") is a kind of cyclic oligosaccharide in which glucose units are mutually bonded by $\alpha(1\rightarrow4)$ glucoside linkage to form a cyclic structure. Cyclodextrins which exist naturally are classified by the number of glucose units into $\alpha$ (six), $\beta$ (seven), and $\gamma$ (eight) types, and many derivatives of them have been synthesized. The cyclodextrins enj oy stable supply of raw materials, are water-soluble, and their properties have been elucidated for the major part thereof. In addition, derivatives of the cyclodextrins can be easily synthesized, and they are biocompatible and biodegradable. Accordingly, the cyclodextrin is a versatile substance that is used in various medicines and foods.

Examples of cyclodextrin include $\alpha$-cyclodextrin, $\beta$-cyclodextrin, and $\gamma$-cyclodextrin. Examples of derivatives of them include modified $\alpha$-, $\beta$-, or $\gamma$-cyclodextrin such as methylated cyclodextrin, maltosylated cyclodextrin, hydroxypropylated cyclodextrin, glucosylated cyclodextrin, sulfobutylated cyclodextrin. As the cyclic molecule, either one selected from these cyclodextrins and their derivatives may be used, or two or more of them may be used in combination. Preferable cyclic molecules in this embodiment are $\alpha$-cyclodextrin and $\gamma$-cyclodextrin.

As these cyclic molecules, those which are commercially available can be used. The cyclodextrin derivatives can be synthesized by introducing a functional group or groups (for example, hydroxypropyl group, glucosyl group, etc.)

to commercially available cyclodextrins by a known method.

The cyclic molecule is preferably contained in the gel preparation in a concentration of 1 mg/mL to 2,000 mg/mL, more preferably in a concentration of 5 mg/mL to 1,000 mg/mL, and further preferably in a concentration of 10 mg/mL to 300 mg/mL. Where two or more kinds of cyclic molecules are used, it suffices that the total amount of them is within the above-mentioned range.

Where the cyclic molecule is α-cyclodextrin, the α-cyclodextrin is preferably contained in the gel preparation in a concentration of 10 mg/mL to 200 mg/mL, more preferably in a concentration of 20 mg/mL to 150 mg/mL, further preferably in a concentration of 30 mg/mL to 145 mg/mL, and most preferably in a concentration of 60 mg/mL to 145 mg/mL.

Where the cyclic molecule is γ-cyclodextrin, the γ-cyclodextrin is preferably contained in the gel preparation in a concentration of 10 mg/mL to 300 mg/mL, more preferably in a concentration of 20 mg/mL to 250 mg/mL, further preferably in a concentration of 50 mg/mL to 235 mg/mL, and most preferably in a concentration of 100 mg/mL to 235 mg/mL.

[Linear molecule]

In the present invention, the linear molecule is a hydrophilic polymer being polyethylene glycol.

In a preferred embodiment of polyethylene glycol, a polyethylene glycol having an average molecular weight of preferably 400 to 35,000, more preferably 2,000 to 20,000, further preferably 4,000 to 20,000 is used as the linear molecule.

The linear molecule is contained in the gel preparation in a concentration of 0.5 mg/mL to 200 mg/mL, preferably in a concentration of 1 mg/mL to 180 mg/mL, and more preferably in a concentration of 2 mg/mL to 160 mg/mL. Where two or more kinds of linear molecules are used, it suffices that the total amount of them is within the above-mentioned range.

The polyethylene glycol is contained in the gel preparation in a concentration of 0.5 mg/mL to 200 mg/mL, preferably in a concentration of 1 mg/mL to 180 mg/mL, and more preferably in a concentration of 2 mg/mL to 160 mg/mL.

Where the cyclic molecule is α-cyclodextrin, the linear molecule is preferably contained in the gel preparation in a concentration of 0.5 mg/mL to 100 mg/mL, more preferably in a concentration of 1 mg/mL to 80 mg/mL, and further preferably in a concentration of 2 mg/mL to 50 mg/mL.

Where the cyclic molecule is γ-cyclodextrin, the linear molecule is preferably contained in the gel preparation in a concentration of 0.5 mg/mL to 200 mg/mL, more preferably in a concentration of 1 mg/mL to 180 mg/mL, and further preferably in a concentration of 2 mg/mL to 160 mg/mL.

[Diluent]

Examples of the diluent include water and buffers.

The buffer is not specifically restricted, and is appropriately selected according to the purpose (for example, the kind of protein). Those buffers which are normally used can be used as usual or after modified appropriately. Specifically, as the buffer, solutions containing conventionally known buffer compositions having a buffering ability can be used without any special limitations. Examples of the buffers usable here include solutions containing an organic acid such as citric acid, succinic acid, tartaric acid, malic acid, etc., salts thereof or the like; amino acids such as glycine, glycylglycine, taurine, arginine, etc.; and solutions containing an inorganic acid such as hydrochloric acid, nitric acid, sulfuric acid, phosphoric acid, boric acid, acetic acid, etc., salts thereof or the like. As a specific buffer, there can be suitably used those known buffers which have a desired pH, without special restrictions. Examples of the specific buffers which can be used include acetate buffers; phosphate buffers (PBS, etc.); citrate buffers; citrate-phosphate buffers; tris buffers such as tris buffer, tris (tris(hydroxymethyl)aminomethane)-HCl buffer (tris-hydrochlorate buffer), tris-glycine buffer, tris-tricine buffer, etc.; amino acid buffers such as glycine-hydrochlorate buffer, glycine-NaOH buffer, glycylglycine-NaOH buffer, glycylglycine-KOH buffer, etc.; borate buffers such as tris-borate buffer, borate-NaOH buffer, borate buffer, etc.; GOOD buffers such as MOPS (3-morpholinopropanesufonic acid) buffer, MOPS-NaOH buffer, HEPES (4-(2-hydroxyethyl)-1-piperazineethanesulfonic acid) buffer, HEPES-NaOH buffer, etc.; and imidazole buffer, etc. The concentration of the buffer is not specifically limited, but may be suitably selected according to the purpose (for example, the kind of protein). For instance, the concentration is preferably 0.1 mM to 200 mM, more preferably 1 mM to 150 mM. Note that the concentration of the buffer in the present invention refers to the concentration (mM) of a buffering agent contained in the buffer. The pH of the buffer is not particularly limited, but may be appropriately selected according to the purpose (for example, the kind of protein) . For instance the pH is preferably in the range of pH 3 to pH 11, more preferably in the range of pH 4 to pH 10, and further preferably in the range of pH 5 to pH 9. The pH of the buffer may be appropriately adjusted by use of an acidic substance such as hydrochloric acid and/or a basic substance such as sodium hydroxide.

Besides, the diluent in this embodiment may further contain additives such as stabilizer, isotonizing agent, pH adjuster, antioxidant, surfactant, solubilizing agent, etc. Specific examples of the additives include amino acids such as arginine, lysine, histidine, aspartic acid, glutamic acid, proline, glycine, alanine, etc.; denaturants such as guanidine hydrochloride, urea, etc.; sugars such as sucrose, glucose, trehalose, mannitol, etc.; electrolytes such as NaCl, KCl, $MgSO_4$, $CaCl_2$, HCl, NaOH, etc.; surfactants such as polysorbate 80, polysorbate 20, etc.; and solubilizing agents such as propylene glycol. The concentrations of these additives are appropriately selected according to the kinds of the additives used. Where electrolytes, denaturants, surfactants or the like are used, they may spoil the stability of the protein when used in a high concentration, and, therefore, care should be taken.

[Protein]

In the present invention, the protein is not specifically restricted so long as it is a pharmacologically acceptable one; preferably, the protein is a protein for medical use (medical protein) . In the gel preparation in this embodiment, the protein is included inside the cyclic molecule and the linear molecule, but the protein exists separately from the cyclic molecule and the linear molecule. In other words, the protein is not a protein which is modified with and united with the cyclic molecule or the linear molecule.

Examples of the protein include antibodies, enzymes, cytokines, and hormones. Specific examples of the protein include therapeutic peptides, human growth hormones, growth hormone-releasing hormone, granulocyte colony-stimulating factor, macrophage colony-stimulating factor, granulocyte macrophage colony-stimulating factor, insulin, lysozyme, asparaginase, leptin, erythropoietin, insulin-like growth factor, tumor necrosis factor, interferon, interleukin, tissue plasminogen activator, urokinase, albumin, monoclonal antibodies, monoclonal antibody fragments, and fusion proteins. Specific examples of the therapeutic peptides include peptide hormones, cancer peptide vaccines, and antibacterial peptides. In the present invention, the proteins in preferred forms are antibodies, enzymes, cytokines, hormones, monoclonal antibodies, monoclonal antibody fragments, and fusion proteins, with more preferred forms being monoclonal antibodies, monoclonal antibody fragments, and fusion proteins.

The protein contained in the gel preparation in the present invention is contained in a concentration of 2.5 mg/mL to 400 mg/mL, in the gel preparation having a gel concentration of 7 wt/vol% to 30 wt/vol%. The protein is preferably contained in the gel preparation, which has a gel concentration of 7 wt/vol% to 30 wt/vol%, in a concentration of 10 mg/mL to 400 mg/mL. The above-mentioned protein concentration is particularly preferably applied to the cases where the protein is an antibody (especially, human IgG antibody) or a cytokine. Even when the gel preparation of the present invention contains the protein (in the included state) in a concentration within the above-mentioned range, the effects of inhibiting aggregation of the protein and stabilizing the protein are exhibited. While the gel preparation of the present invention can stably contain the protein in a high concentration within the above-mentioned range, the effectiveness of the gel preparation of the present invention is especially exhibited with the protein concentration set within the above-mentioned range.

The protein contained in the gel preparation of the present invention preferably has a molecular weight of not less than 3,000 Da, more preferably not less than 20,000 Da, further preferably not less than 30,000 Da, and most preferably not less than 40,000 Da. While there is no upper limit to the protein contained in the gel preparation, the molecular weight is preferably not more than 1,000,000 Da. Where the molecular weight of the protein is within the above-mentioned range, aggregation of the protein contained (in the included state) in the gel preparation of the present invention is inhibited effectively.

Many of proteins are susceptible to aggregation in preparations, due to their properties. Besides, as the concentration of a protein in a preparation is higher, the protein is more likely to aggregate in the preparation. The gel preparation of the present invention can stabilize a protein in the gel preparation, even in the case where it contains (in an included state) a protein in a high concentration or a protein susceptible to aggregation.

A preferred form of the gel preparation in this embodiment is a gel preparation in which the cyclic molecule is contained in a concentration of 10 mg/mL to 300 mg/mL, the liner molecule is contained in a concentration of 0.5 mg/mL to 200 mg/mL, and the protein is contained in a concentration of 0.1 mg/mL to 400 mg/mL. When the concentrations are within such respective ranges, the gel includes (contains in an included state) the protein effectively, whereby the protein is effectively inhibited from aggregating.

[Method of producing gel preparation]

The gel preparation according to this embodiment is thixotropic, so that the gel is fluidized when stirred, even after the gel is prepared. The method for preparing the gel preparation is not particularly limited, and the cyclic molecule, the linear molecule, the protein, and the diluent may be added in any order so long as they are mixed with one another. For example, the gel preparation in this embodiment may be prepared by dissolving the cyclic molecule (or the linear molecule) and the protein in the diluent, followed by adding the linear molecule (or the cyclic molecule) thereto and mixing them, to form the gel preparation. Alternatively, the gel preparation may be prepared by mixing the cyclic molecule, the linear molecule, and the diluent to form a gel, followed by adding the protein to the gel and mixing them. Further, the gel preparation may be prepared by simultaneously adding the cyclic molecule, the linear molecule, and the protein to the diluent, followed by mixing.

A preferable method of producing the gel preparation of the present invention includes a step of mixing a first gelling agent with a second gelling agent, wherein the first gelling agent contains the cyclic molecule, the protein, and the diluent, and the second gelling agent contains the linear molecule, the protein, and the diluent.

The temperature at which the cyclic molecule, the linear molecule, the protein, and the diluent are mixed is not particularly limited so long as the protein is not decomposed or altered at that temperature. Besides, the method for mixing the components is not specifically restricted so long as the components are mixed to form a uniform gel preparation.

[Use of the gel preparation]

As aforementioned, the gel preparation according to the present invention permits an appropriate selection of a protein according to the purpose (the kind of a disease to be treated), and can therefore be used as a therapeutic drug for various diseases. The method of administering the gel preparation is not specifically restricted, and the gel preparation can be used as a drug for parenteral administration, especially a drug for subcutaneous administration, for prevention, treatment or therapy of diseases. While the gel preparation of the present invention is preferably a drug for subcutaneous administration, an arbitrary route of administration can be selected such as, for example, agent for lesional local external administration, lesional local injection, nose drop, eye drop, eye ointment, auristillae, suppository, intravenous injection, endermic injection, intramuscular injection, inhalant, drug for perlingual administration, drug for percutaneous absorption, etc. Since the gel preparation of the present invention is thixotropic, the gel preparation can be delivered from inside a device to a target place by exerting a pressure, without needing any complicated operation. Therefore, the gel preparation filled in a cylinder of a syringe, for example, can be directly administered subcutaneously as an injection; accordingly, the gel preparation is preferably a drug for subcutaneous administration.

The dose of the gel preparation of the present invention is not particularly limited, and can be appropriately selected according to the purpose (the kind of a disease to be treated) and the extent (condition) thereof.

[Medical kit]

A further embodiment of the present invention is a medical kit comprising a gel preparation according to the present invention, and an administering device for administering the gel preparation.

The administering device is preferably a syringe (a cylinder and an injection needle).

In the medical kit according to the present invention, the gel preparation may be sealed in a vial or the like, or may preliminarily be filled in a cylinder of a syringe.

Examples

The effects of the present invention will be described referring to the following Examples and Comparative Examples. The technical scope of the present invention, however, is not limited to the following Examples. Note that the following operations were conducted at room temperature (25°C), unless specified otherwise.

[Example 1: Measurement of gel concentration]

In 0.5 L of distilled water were dissolved 2.92 g of NaCl, 0.50 g of disodium hydrogen phosphate, 1.43 g of sodium dihydrogen phosphate dihydrate, 2.63 g of L-arginine hydrochloride, and 5.0 g of sucrose, to form a solution A.

Either $\alpha$-cyclodextrin or $\gamma$-cyclodextrin was dissolved in the solution A by warming on a water bath at around 60°C, followed by cooling to room temperature. Next, polyethylene glycol (PEG) 20000 was added to the resulting solution according to compositions set forth in Table 1 below, followed by mixing in a microtube and still-standing at 4°C for not less than 12 hours, to prepare gels 1 to 59 of formulations 1 to 59.

Besides, gels 60 to 93 of formulations 60 to 93 were prepared according to compositions set forth in Table 2 below, using polyethylene glycols having the respective average molecular weights. The average molecular weights of the PEGs used, the kinds of cyclodextrin and the formulation numbers are set forth in Table 2 below.

The blank columns in Table 1 and Table 2 below means the absence of the relevant ingredient.

Note that as PEG there were used PEG20000 (product name: Polyethylene glycol 20,000, produced by Wako Pure Chemical Industries, Ltd.), PEG400 (product name: Polyethylene glycol 400, produced by Kanto Chemical Co., Ltd.), PEG600 (product name: Polyethylene glycol 600, produced by Kanto Chemical), PEG1000 (product name: polyethylene glycol 1000, produced by Kanto Chemical), PEG2000 (product name: Polyethylene glycol 2000, produced by Merck), PEG3000 (product name: polyethylene glycol 3000, produced by Merck), PEG4000 (product name: polyethylene glycol 4000, produced by Kanto Chemical), PEG6000 (product name: Polyethylene glycol 6000, produced by SERVA Electrophotoresis GmbH), PEG8000 (product name: Polyethylene glycol, MW 8000, produced by MP Biomedicals LLC), PEG10000 (product name: Polyethylene glycol 10000, produced by Merck), and PEG35000 (product name: Polyethylene glycol 35000, produced by Merck). Besides, as α-cyclodextrin, there were used an α-cyclodextrin with a product name Celldex (registered trademark) A-100 (produced by Nihon Shokuhin Kako Co., Ltd.) and a γ-cyclodextrin with a product name DexyPearl γ-100 (produced by Ensuiko Sugar Refining Co., Ltd.).

Next, for the gels obtained, gel concentration (wt/vol%) was measured. The microtubes filled with the above-mentioned gels 1 to 93 were set on a centrifuge, centrifugation (4°C) was conducted at 14,000 × G for 45 minutes to separate each gel into gel form solids and a supernatant, the supernatant thus separated was removed, and the solids were dried at 105°C for five hours. The weight (g) of each of the solid matters (dried matters) thus left was measured. Using the weight and the gel volume, gel concentration (wt/vol%) was calculated according to the following formula [1], the results being set forth in Table 1 and Table 2.

[Mathematical formula 2]

$$\text{Gel concentration } (wt/vol\%) = \frac{Solids\ weight\ of\ gel\ (g)}{Volume\ of\ gel\ (mL)} \times 100 \qquad [1]$$

Solids weight of gel (g): Weight of precipitate (solids) obtained by centrifugation of gel (g)

[Table 1]

Table 1

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| α-Cyclodextrin(mg/mL) | 53.3 | 64.4 | 64.4 | 64.4 | 72.5 | 72.5 | 72.5 | 72.5 | 72.5 | 128.9 | 128.9 | 128.9 | | | | | | |
| γ-Cyclodextrin(mg/mL) | | | | | | | | | | | | | 106.7 | 141.8 | 141.8 | 141.8 | 154.7 | 154.7 |
| PEG20000(mg/mL) | 11.1 | 11.1 | 22.2 | 44.4 | 4.4 | 6.7 | 11.1 | 22.2 | 44.4 | 4.4 | 6.7 | 11.1 | 22.2 | 22.2 | 88.9 | 155.6 | 4.4 | 8.9 |
| Gel concentration(wt/vol%) | 0.2 | 5.5 | 7.2 | 8.9 | 5.3 | 6.1 | 7.8 | 8.7 | 10.3 | 7.4 | 9.1 | 13.6 | 5.4 | 12.4 | 18.7 | 20.6 | 7.5 | 12.9 |

| | 19 | 20 | 21 | 22 | 23 | 24 | 25 | 26 | 27 | 28 | 29 | 30 | 31 | 32 | 33 | 34 | 35 | 36 |
|---|---|---|---|---|---|---|---|---|---|---|---|---|---|---|---|---|---|---|
| α-Cyclodextrin (mg/mL) | | | | | | | | | | | | | | | | | | |
| γ-Cyclodextrin (mg/mL) | 154.7 | 154.7 | 154.7 | 154.7 | 154.7 | 154.7 | 154.7 | 180.4 | 180.4 | 180.4 | 180.4 | 180.4 | 180.4 | 180.4 | 206.2 | 206.2 | 206.2 | 206.2 |
| PEG20000(mg/mL) | 13.3 | 17.8 | 22.2 | 33.3 | 44.4 | 88.9 | 133.3 | 4.4 | 8.9 | 13.3 | 17.8 | 22.2 | 33.3 | 44.4 | 4.4 | 8.9 | 13.3 | 17.8 |
| Gel concentration (wt/vol%) | 12.5 | 15.1 | 16.2 | 17.1 | 17.9 | 20.5 | 23.4 | 10.6 | 14.9 | 18.0 | 18.5 | 19.5 | 20.3 | 20.8 | 12.6 | 19.8 | 22.6 | 22.0 |

| | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|
| α-Cyclodextrin (mg/mL) | | | | 87.0 | 104.4 | 116.0 | 116.0 | 116.0 | 116.0 | 87.0 | 110.2 | 130.5 | 103.1 | | | | | |
| γ-Cyclodextrin (mg/mL) | 206.2 | 206.2 | 206.2 | | | | | | | 46.4 | | | | 139.2 | 167.0 | 185.6 | 185.6 | 185.6 |
| PEG20000(mg/mL) | 22.2 | 33.3 | 44.4 | 10 | 10 | 10.0 | 5.0 | 2.0 | 1.0 | 12.5 | 10 | 5.0 | 8.9 | 20 | 20 | 20.0 | 10.0 | 5.0 |
| Gel concentration (wt/vol%) | 23.2 | 23.2 | 24.1 | 8.4 | 9.9 | 10.9 | 7.3 | 4.1 | 2.3 | 11.3 | 10.4 | 7.9 | 10.2 | 11.2 | 14.9 | 17.3 | 14.2 | 8.7 |

| | 55 | 56 | 57 | 58 | 59 |
|---|---|---|---|---|---|
| α-Cyclodextrin(mg/mL) | | | | | |
| γ-Cyclodextrin(mg/mL) | 185.6 | 185.6 | 176.3 | 206.2 | 159.1 |
| PEG20000(mg/mL) | 2 | 1.0 | 20 | 11.1 | 11.4 |
| Gel concentration(wt/vol%) | 4.0 | 2.2 | 16.1 | 21.2 | 13.3 |

[Table 2]

Table 2

| | 60 | 61 | 62 | 63 | 64 | 65 | 66 | 67 | 68 | 69 | 70 | 71 | 72 | 73 | 74 | 75 | 76 | 77 |
|---|---|---|---|---|---|---|---|---|---|---|---|---|---|---|---|---|---|---|
| α-Cyclodextrin(mg/mL) | 128.9 | 128.9 | 128.9 | 128.9 | 128.9 | 128.9 | 128.9 | 128.9 | 128.9 | 128.9 | | | | | | | | |
| γ-Cyclodextrin(mg/mL) | | | | | | | | | | | 206.2 | 206.2 | 206.2 | 206.2 | 206.2 | 206.2 | 206.2 | 206.2 |
| PEG400(mg/mL) | 11.1 | | | | | | | | | | 22.2 | | | | | | | |
| PEG600(mg/mL) | | 11.1 | | | | | | | | | | 22.2 | | | | | | |
| PEG1000(mg/mL) | | | 11.1 | | | | | | | | | | 22.2 | | | | | |
| PEG2000(mg/mL) | | | | 11.1 | | | | | | | | | | 22.2 | | | | |
| PEG3000(mg/mL) | | | | | 11.1 | | | | | | | | | | 22.2 | | | |
| PEG4000(mg/mL) | | | | | | 11.1 | | | | | | | | | | 22.2 | | |
| PEG6000(mg/mL) | | | | | | | 11.1 | | | | | | | | | | 22.2 | |
| PEG8000(mg/mL) | | | | | | | | 11.1 | | | | | | | | | | 22.2 |
| PEG10000(mg/mL) | | | | | | | | | 11.1 | | | | | | | | | |
| PEG20000(mg/mL) | | | | | | | | | | | | | | | | | | |
| PEG35000(mg/mL) | | | | | | | | | | 11.1 | | | | | | | | |
| Gel concentration(wt/vol%) | 8.1 | 10.7 | 11.0 | 11.2 | 11.2 | 11.0 | 12.4 | 12.7 | 11.2 | 11.9 | 15.0 | 16.3 | 17.4 | 18.2 | 17.7 | 18.0 | 18.2 | 18.8 |

| | 78 | 79 | 80 | 81 | 82 | 83 | 84 | 85 | 86 | 87 | 88 | 89 | 90 | 91 | 92 | 93 |
|---|---|---|---|---|---|---|---|---|---|---|---|---|---|---|---|---|
| α-Cyclodextrin(mg/mL) | | | 116.0 | | 116.0 | 116.0 | 116.0 | 116.0 | | | | | 116.0 | 116.0 | | |
| γ-Cyclodextrin(mg/mL) | 206.2 | 206.2 | | 185.6 | | | | | 185.6 | 185.6 | 185.6 | 185.6 | | | 185.6 | 185.6 |
| PEG400(mg/mL) | | | | | 1.0 | 2.5 | 5.0 | 7.5 | 2.0 | 5.0 | 10.0 | 15.0 | | | | |
| PEG600(mg/mL) | | | | | | | | | | | | | | | | |
| PEG1000(mg/mL) | | | | | | | | | | | | | | | | |
| PEG2000(mg/mL) | | | | | | | | | | | | | | | | |
| PEG3000(mg/mL) | | | | | | | | | | | | | | | | |
| PEG4000(mg/mL) | | | | | | | | | | | | | 1.0 | 2.5 | 5.0 | 10.0 |
| PEG6000(mg/mL) | | | | | | | | | | | | | | | | |
| PEG8000(mg/mL) | | | | | | | | | | | | | | | | |
| PEG10000(mg/mL) | 22.2 | | | | | | | | | | | | | | | |
| PEG20000(mg/mL) | | | | | 9.0 | 7.5 | 5.0 | 2.5 | 18 | 15 | 10 | 5 | 9.0 | 7.5 | 15.0 | 10.0 |
| PEG35000(mg/mL) | | 22.2 | 10.0 | 20.0 | | | | | | | | | | | | |
| Gel concentration(wt/vol%) | 19.2 | 23.0 | 11.6 | 18.3 | 10.6 | 11.0 | 9.5 | 6.8 | 17.0 | 16.6 | 16.4 | 13.0 | 10.7 | 9.5 | 17.3 | 17.2 |

As shown in the results set forth in Table 1 and Table 2, a gel concentration of not less than 4 wt/vol% was obtained with 64.4 mg/mL to 130.5 mg/mL of α-cyclodextrin, or 106.7 mg/mL to 206.2 mg/mL of γ-cyclodextrin, or 133.4 mg/mL in total of α-cyclodextrin and γ-cyclodextrin, and with a PEG20000 concentration in the range of 2.0 mg/mL to 155.6 mg/mL and a PEG average molecular weight in the range of 400 to 35,000.
Not that in the following Examples 2 and 4 to 32, the above-mentioned formulations 1 to 93 with a predetermined amount of a protein contained therein are made to be gel preparations.

[Example 2: Physical properties of gel preparations]

(1) Preparation of gel preparation

Gel preparations 2-12 and 2-37 of formulations 12 and 37 in Example 1 were prepared in which 115.6 mg/mL of human IgG was contained. The gel preparation 2-12 of the formulation 12 was prepared as follows. In the solution A was dissolved 145 mg of α-cyclodextrin by warming on a water bath at around 60°C, followed by cooling to room temperature. Next, 0.13 g of human IgG was dissolved in the resulting solution at room temperature (25°C), to prepare a cyclodextrin/human IgG solution, which was subjected to filtration disinfection by use of a 0.22 μm filter. Besides, 1.0 g of PEG20000 was dissolved in 10 mL of the solution A, to prepare a PEG solution, which was subjected to filtration disinfection by use of a 0.22 μm filter. After 0.8 mL of the cyclodextrin/human IgG solution and 0.1 mL of the PEG solution were mixed with each other at room temperature (25°C), the resulting mixture was left to stand at 4°C for not less than 12 hours, to prepare the gel preparation 2-12 containing the human IgG in an included state. The gel preparation 2-37 was prepared in the same manner as the gel preparation 2-12. These gel preparations were each washed with 100 μL of water, and dried under reduced pressure at room temperature (25°C) for at least three days.

(2) Calculation of number of threading ($^1$H-NMR measurement)

Solid samples 2-12a and 2-37a obtained upon drying in (1) above were each dissolved in DMS-$d_6$, and subjected to measurement of $^1$H-NMR spectrum. FIGS. 1A and 1B show the spectra of the solid samples 2-12a and 2-37a, respectively. Integrated values of proton peaks of cyclodextrin and PEG of the spectrum in FIG. 1A and those of the spectrum in FIG. 1B were compared (cyclodextrin: a peak value of 4.8 ppm due to anomeric proton; PEG: a peak value of 3.5 ppm due to ethyl proton). On the basis of the comparison, it was considered that about 113 α-cyclodextrin molecules and about 95 γ-cyclodextrin molecules were respectively threaded on (penetrated by) a single PEG chain. Theoretically, about 220 α-cyclodextrin molecules and about 110 γ-cyclodextrin molecules can be threaded on (penetrated by) a single PEG chain having an average molecular weight of 20,000. Therefore, the threading rates of the α- and γ-cyclodextrins were about 51.5% and 86.2%, respectively.

(3) Analysis of crystal structure (Powder X-ray diffraction measurement)

Powder X-ray diffraction measurement was conducted for (g) the solid samples 2-12a and 2-37a of human IgG-containing gel preparations produced in (1) above, as well as comparative solid samples consisting of (a) cyclodextrin (CyD) alone, (b) PEG alone, (c) human IgG alone, (d) a PEG/CyD mixture, (e) a PEG/CyD/human IgG mixture, and (f) a human IgG-noncontaining gel preparation. Note that the comparative solid samples were samples obtained by mixing the respective solids in predetermined mixing ratios (corresponding respectively to the mixing ratios of formulation 12 and formulation 37). The solid sample of (f) human IgG-noncontaining gel preparation was a sample prepared in the same manner as the preparation of the gel preparations in (1) above, except that the human IgG was not contained in the gel preparation. The results of powder X-ray diffraction for the solid samples 2-12a and 2-37a of the gel preparations 2-12 and 2-37 are shown in FIG. 2A and FIG. 2B, respectively. Besides, powder X-ray diffraction peak values and assignment of indices for the solid samples 2-12a and 2-37a of the gel preparations 2-12 and 2-37 are shown in FIG. 3A and FIG. 3B, respectively.
In the α-cyclodextrin system, the physical mixture with PEG showed a diffraction pattern of a cage structure, like the α-cyclodextrin used alone. On the other hand, the solid sample 2-12a of the gel preparation 2-12 (formulation 12) containing the human IgG gave characteristic peaks at $2\theta$ = 7.52, 13.00, 19.82, and 22.53 degrees, showing a diffraction pattern of a tubular structure, like the solid sample of the gel preparation (gel) to which the human IgG had not been added. Similarly, in the γ-cyclodextrin system, the γ-cyclodextrin alone and the physical mixture with PEG showed a diffraction pattern of a cage structure. On the other hand, the solid sample 2-37a of the gel preparation 2-37 (formulation 37) containing the human IgG gave characteristic peaks at $2\theta$ = 7.36, 14.79, 16.55, and 22.05 degrees, showing a diffraction pattern of a tubular structure, like the solid sample of the gel preparation (gel) of the same formulation.
Furthermore, in order to elucidate the details of the manner in which the crystal is packed, the diffraction patterns for the gel preparations containing the α- or γ-cyclodextrin were presumed to be those of a hexagonal system and a tetragonal system, respectively, and assignment of indices for each of diffraction lines was conducted. As a result, in regard to each of plane indices (hkl), good agreement between $d_{obs}$ (observed value) and $d_{cal}$ (calculated value) was obtained. This showed that the human IgG-containing gel preparations 2-12a and 2-37a are a hexagonal system tubular structure and a tetragonal system tubular structure, respectively. In addition, from the peak areas of the solid samples 2-12a and 2-37a, the degrees of crystallization of the samples are considered to be 49.32% and 22.60%, respectively.

(4) FT-IR measurement

FT-IR measurement was conducted for (c) the solid samples 2-12a and 2-37a produced in (1) above as the IgG-containing gel preparations, as well as comparative solid samples consisting of (a) human IgG alone, and (b) cyclodextrin alone. The result of FT-IR measurement for the solid sample 2-12a and that for the solid sample 2-37a are shown in FIG. 4A and FIG. 4B, together with the results of FT-IR measurement for the comparative solid samples.

The OH-derived broad peak at 4,200 $cm^{-1}$ to 3,000 $cm^{-1}$ observed in the spectrum for the cyclodextrin alone was slightly shifted to the higher wavenumber side in spectra for the human IgG-containing gel preparations 2-12 and 2-37. This is considered to be attributable to the formation of hydrogen bonds between the OH groups of the adjacent cyclodextrin molecules attendant on gel formation.

From the results of (2) to (4) above, it was confirmed that in the human IgG-containing gel preparations, a cyclodextrin-PEG composite had been formed.

[Example 3: Variations in gel's yield value and viscosity with time]

The gels 12 and 37 having the compositions of the formulations 12 and 37 in Example 1, in an amount of 1 mL, were subjected to measurement of yield value and viscosity (25°C) by use of a rheometer R/S plus (produced by Brookfield) and a cone/plate spindle with a diameter of 2.5 cm. Note that the viscosity measurement involved measurement, every one second, of variations in the viscosity of the gel brought about when a stress at a share rate (100/s) is exerted on the gel.

Measurement results of yield value for the gels 12 and 37 are set forth in Table 3, and variations in gel viscosity for the gels are shown in FIGS. 5A and 5B, respectively. These results show that the gels 12 and 37 have yield values, and their viscosities are lowered with time when a stress is exerted thereon.

[Table 3]

Table 3. Yield value of gel

| | Gel 12 | Gel 37 |
|---|---|---|
| Yield value(Pa) | 1175.3 | 4709.2 |

[Example 4: Confirmation of thixotropy in gel preparations]

The gel preparations 4-12 and 4-37 of formulations 12 and 37 containing 115.6 mg/mL of human IgG, each in an amount of 1 mL, were subjected to measurement of viscosity (37°C) by use of TVE20H (produced by Toki Sangyo Co., Ltd.). Note that the gel preparations 4-12 and 4-37 were prepared by the same method as in (1) of Example 2. As for the viscosity measurement, a variation in the viscosity of the gel brought about when a stress at a share rate (100/s) is exerted on the gel was measured after one minute, two minutes, three minutes, four minutes, and five minutes from the start of measurement. Further, after one hour and after three hours from the first viscosity measurement, the viscosity was again measured under the same conditions.

The results are shown in FIGS. 6A and 6B. After one hour from the first measurement, neither of the gel preparations of two formulations showed a recovery in viscosity. When measured after three hours from the first measurement, the gel preparations showed a recovery in viscosity. These results show that the IgG-containing gel preparations are thixotropic.

[Example 5: Activity retention rate of gel preparation -1]

Gel preparations 5-40 and 5-50 of formulations 40 and 50 each containing 10.0 mg/mL of soluble TNFa/LTa receptor fusion protein were each prepared in an amount of 250 μL in a microtube, in the same manner as in the preparing method in (1) of Example 2. Each of the gel preparations was preserved in a cold storage for seven days, and 1 mL of the solution A was added thereto, followed by gentle shaking to disintegrate the gel preparation and by centrifugation. Thereafter, the supernatants obtained were each filtered through a membrane filter. For soluble TNFa/LTa receptor fusion protein in the eluates, inhibitory activity on TNFα was measured by an assay based on the use of cells. In the measurement, a 10 mg/mL solution of the soluble TNFa/LTa receptor fusion protein was used as a control, and the measurement was conducted with N = 3.

The results are set forth in Table 4 below. It was made clear that the soluble TNFa/LTa receptor fusion proteins contained (in an included state) in the gel preparations retained their activity at rates of 109.9% and 117.1%, respectively.

[Table 4]

Table 4. Activity retention rate of soluble TNFα/LTα receptor fusion protein contained (in included state) in gel preparation

| | Gel preparation 5-40 | Gel preparation 5-50 |
|---|---|---|
| 1 | 99.8 | 115.0 |
| 2 | 109.6 | 116.9 |
| 3 | 120.3 | 119.4 |
| Average | 109.9 | 117.1 |

[Example 6: Activity retention rate of gel preparations -2]

Gel preparations 6-41 and 6-51 of formulations 41 and 51 each containing 7.0 mg/mL of antihuman IgE antibody were each prepared in an amount of 250 μL in a microtube, in the same manner as in the preparing method in (1) of Example 2, while using water in place of the solution A. Each of the gel preparations was preserved in a cold storage for five days, and was then subjected to disintegration of gel preparation by microspatula, followed by centrifugation. Thereafter, the supernatants obtained were each filtered through a membrane filter. For the antihuman IgE antibody in each of the supernatants, inhibitory activity on human IgE binding was measured by the following method. Note that this experiment was conducted with N = 2.

I. The absorbance of each supernatant at 280 nm was measured by a UV spectrophotometer (model V-650, produced by JASCO Corporation). From the absorbance value, remaining rate of the antihuman IgE antibody contained in the supernatant was calculated.

II. After the protein concentration was calibrated on the basis of the results of I above, human IgE binding inhibition activity of the antihuman IgE antibody in the supernatant was measured, as antihuman IgE antibody activity, by an ELISA method while using a plate on which FcεR1 receptor was provided as a solid phase.

As a control, an antihuman IgE antibody solution obtained by dissolving antihuman IgE antibody in water to obtain a concentration of 7.0 mg/mL was treated in the same manner as above, exclusive of the operation of disintegrating the gel preparation by the microspatula. The activity retention rate of the protein contained in the supernatant was calculated, based on the activity possessed by the antihuman IgE antibody solution used as the control.

The results are set forth in Table 5. It was found that the antihuman IgE antibodies contained (in an included state) in the gel preparations retained their activity at rates of 96.3% and 104.1%, respectively.

[Table 5]

Table 5. Activity retention rate of antihuman IgE antibody contained (inincluded state) in gel preparation and released therefrom (%)

| | Gel preparation 6-41 | Gel preparation 6-51 |
|---|---|---|
| 1 | 93.6 | 104.7 |

(continued)

| | Gel preparation 6-41 | Gel preparation 6-51 |
|---|---|---|
| 2 | 99.0 | 103.5 |
| Average | 96.3 | 104.1 |

[Example 7: Evaluation of resistance to pushing-out of gel preparation from syringe]

Gel preparations 7-42(8), 7-42(40), 7-42(80) of formulation 42 and gel preparations 7-52(8), 7-52(40), 7-52(8) of formulation 52 respectively containing 8.0, 40.0, and 80.0 mg/mL of human IgG, and gel preparations of formulation 43 and formulation 53 both containing 80 mg/mL of human IgG were each prepared in an amount of 0.5 mL in a 1-mL tuberculin syringe (produced by Terumo Corporation) in the same manner as in the preparing method in (1) of Example 2, while using Phosphate buffered saline (PBS, pH 7.4) in place of the solution A. After the gel preparations were preserved in a cold storage for seven days, resistance to pushing-out of gel preparation at the time of pushing out the gel preparation from a syringe at a rate of 228 mm/min was measured on a desktop autograph (produced by Shimadzu Corporation), using a 25G injection needle (produced by Terumo Corporation) for the gel preparations of formulations 42 and 52 and using a 27G injection needle (produced by Terumo Corporation) for the gel preparations of formulations 43 and 53. The values of sliding resistance are represented by an average resistance value (N) over a period from 4.0 sec to 8.0 sec after the start of pushing-out in which period the resistance value is substantially constant. Note that this experiment was conducted with N = 2.

The results are set forth in Tables 6-1, 6-2, and 6-3. It was found that the pushing-out resistance tends to depend on the protein concentration. The gel preparations containing 80 mg/mL of the human IgG showed a pushing-out resistance of 7.1 N for the formulation 42 and 9.9 N for the formulation 52. Thus, it is confirmed that the gel preparations containing 80 mg/mL of the human IgG can be manually pushed out of a syringe.

[Table 6]

Table 6-1. Pushing-out resistance of gel preparations 7-42 when 1 mL syringe and 25G needle were used (N)

| | Gel preparation 7-42 (80) | Gel preparation 7-42 (40) | Gel preparation 7-42 (8) |
|---|---|---|---|
| 1 | 6.4 | 6.0 | 6.3 |
| 2 | 7.8 | 6.8 | 5.6 |
| Average | 7.1 | 6.4 | 5.9 |

Table 6-2. Pushing-out resistance of gel preparations 7-52 when 1 mL syringe and 25G needle were used (N)

| | Gel preparation 7-52 (80) | Gel preparation 7-52 (40) | Gel preparation 7-52 (8) |
|---|---|---|---|
| 1 | 10.3 | 6.2 | 6.9 |
| 2 | 9.4 | 6.7 | 8.8 |
| Average | 9.4 | 6.4 | 7.9 |

Table 6-3. Pushing-out resistance of gel preparations 7-43 and 7-53 when 1 mL syringe and 27G needle were used (N)

| | Gel preparation 7-43 (80) | Gel preparation 7-53 (80) |
|---|---|---|
| 1 | 7.1 | 9.1 |
| 2 | 6.6 | 9.7 |
| Average | 6.9 | 9.4 |

[Example 8: Inhibitory effect on aggregation induced by agitation stress 1]

Gel preparations 8-12 and 8-37 of formulation 12 and 37 each containing 115.6 mg/mL of human IgG were each prepared in an amount of 100 μL in a microtube, in the same manner as in the preparing method in (1) of Example 2. Next, with the microtubes set on a shaking apparatus, shaking at 500 rpm was conducted at room temperature (25°C) for one week, thereby exerting an agitation stress on the gel preparations. After the loading with the agitation stress, 1 mL of the solution A was added to each gel preparation, and the admixture was gently shaken until the gel structure disappeared on a visual observation basis, thereby disintegrating the gel, and the human IgG was eluted. The resulting human IgG eluate was centrifuged to precipitate aggregates, and then the absorbance of the supernatant at 280 nm was measured on a UV spectrophotometer (model V-630, produced by JASCO Corporation). From the absorbance value, the amount of the human IgG contained in the eluate (human IgG remaining rate) was calculated. Note that this experiment was conducted with N = 6, and the human IgG remaining rate was expressed based on the starting amount of human IgG.

As a control group for the human IgG-containing gel preparations 8-12 and 8-37, there were prepared a preparation obtainable by removing cyclodextrin from the gel preparation 8-37 ("PEG + IgG"), preparations obtainable by removing PEG20000 from the gel preparations 8-12 and 8-37 ("α-CyD + IgG" and "γ-CyD + IgG"), and a preparation obtainable by removing cyclodextrin and PEG20000 from the gel preparation 8-37 ("IgG solution"). These preparations in the control group were treated in the same manner as the human IgG-containing gel preparations 8-12 and 8-37.

The measurement results of the remaining rate of human IgG against loading with agitation stress are shown in FIG. 7. By the agitation stress, the amounts of human IgG in the solutions in the control group were reduced to about 60% of the starting amount. On the other hand, the human IgG contained (in an included state) in the gel preparations showed a remaining rate close to 100%. From these results, it was verified that in the high-concentration human IgG solutions, the gel preparations have an effect of stabilizing the human IgG against agitation-induced aggregation.

[Example 9: Inhibitory effect on aggregation induced by agitation stress 2]

Gel preparations 9-2, 9-4, 9-5, 9-6, 9-11, 9-12, 9-18, 9-26, 9-33, and 9-37 of formulations 2, 4, 5, 6, 11, 12, 18, 26, 33, and 37 containing 57.8 mg/mL to 115.6 mg/mL of human IgG were each prepared in an amount of 100 μL in a microtube, in the same manner as in the preparing method in (1) of Example 2. Next, in the same manner as in Example 8, each gel preparation was loaded with an agitation stress, then the IgG was eluted, and the amount of human IgG contained in the eluate was calculated. Note that this experiment was conducted with N = 3, and the remaining rate of human IgG was expressed based on the starting amount. Besides, as a control, a human IgG solution (expressed as "IgG") containing 115. 6 mg/mL of human IgG in 100 μL of the solution A was subjected to the same process as that for the gel preparations. Note that the gel preparations and the respective amounts of human IgG contained in the gel preparations are as follows.

Gel preparations 9-2 and 9-4: Human IgG, 57.8 mg/mL
Gel preparations 9-5 and 9-6: Human IgG, 65.0 mg/mL
Gel preparations 9-11 and 9-12: Human IgG, 115.6 mg/mL
Gel preparation 9-18: Human IgG, 86.7 mg/mL
Gel preparation 9-26: Human IgG, 101.1 mg/mL
Gel preparations 9-33 and 9-37: Human IgG, 115.6 mg/mL

The results are shown in FIG. 8. The amount of human IgG in the human IgG solution was reduced to or below 60% by the agitation stress. On the other hand, the human IgG contained (in an included state) in the gel preparation of each formulation showed a high remaining rate as compared with that in the human IgG solution. Thus, it was made clear that the gel preparations prepared with the cyclodextrin and PEG in the concentrations according to the respective formulations all have an effect of stabilizing the human IgG against agitation-induced aggregation.

[Example 10: Inhibitory effect on aggregation induced by agitation stress 3]

Gel preparations 10-2, 10-4, 10-5, 10-6, 10-11, 10-12, 10-16, 10-18, 10-25, 10-26, 10-33, and 10-37 of formulations 2, 4, 5, 6, 11, 12, 16, 18, 25, 26, 33, and 37 each containing 30.5 mg/mL to 44.4 mg/mL of human IgG were each prepared in an amount of 100 μL in a microtube, in the same manner as in the preparing method in (1) of Example 2, while using Phosphate buffered saline (PBS, pH 7.4) in place of the solution A. Next, the microtubes were set on a shaking apparatus, and shaking at 500 rpm was conducted at room temperature (25°C) for five days, to give an agitation stress to the gel preparations. After the loading with the agitation stress, 1 mL of PBS was added to each gel preparation, and the admixture was gently shaken until the gel structure disappeared on a visual observation basis, thereby disintegrating the gel, and the human IgG was eluted. Each of the human IgG eluates was centrifuged, to precipitate aggregates, and the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 2, and the remaining rate of human IgG was expressed based on the starting amount. Besides, as a control, a human IgG solution prepared by dissolving human IgG in PBS so as to obtain a concentration of 44.4 mg/mL was subjected to the same process as that for the gel preparations. Note that the gel preparations and the respective amounts of human IgG contained in the gel preparations are as follows.

Gel preparations 10-2, 10-5, 10-6, 10-11, 10-12, 10-18, 10-26, 10-33, and 10-37: Human IgG, 44.4 mg/mL
Gel preparation 10-4: Human IgG, 38.9 mg/mL
Gel preparation 10-16: Human IgG, 30.5 mg/mL
Gel preparation 10-25: Human IgG, 33.3 mg/mL

The results are shown in FIG. 9. The remaining rate of human IgG in the eluate of the control (IgG solution) was about 20%. On the other hand, the human IgG contained (in an included state) in the gel preparations obtained by use of α-cyclodextrin and the gel preparations obtained by use of γ-cyclodextrin remained in the eluates in amounts of 86.9% to 97.1% based on the starting amount. Thus, the human IgG contained (in the included state) in the gel preparations was found to have been remarkably stabilized against agitation stress.

[Example 11: Inhibitory effect on aggregation induced by thermal stress]

Gel preparations 11-12 and 11-37 of formulations 12 and 37 each containing 115.6 mg/mL of human IgG were each prepared in an amount of 0.5 mL in a microtube, in the same manner as in the preparing method in (1) of Example 2. Next, 0.5 mL of each gel preparation was heated on a hot bath at 60°C for 15 minutes, so as to give a thermal stress to the gel preparation. After the loading with the thermal stress, 30 mL of the solution A was added to each gel preparation, and the admixture was gently shaken until the gel structure disappeared on a visual observation basis, to disintegrate the gel, and the human IgG was eluted. Each of the human IgG eluates obtained was centrifuged, to precipitate aggregates, and the absorbance of the supernatant at 280 nm was measured on a UV spectrophotometer (model V-630, produced by JASCO Corporation). From the absorbance values, the amounts of human IgG contained in the supernatants were calculated. Note that this experiment was conducted with N = 4 to 8, and the remaining rate of human IgG was expressed based on the starting amount.

Besides, as controls, there were prepared a preparation obtainable by removing cyclodextrin from the gel preparation 11-37 ("PEG + IgG"), preparations obtainable respectively by removing PEG20000 from the gel preparations 11-12 and 11-37 ("α-CyD + IgG" and "γ-CyD + IgG"), and a preparation obtainable by removing cyclodextrin and PEG20000 from the gel preparation 11-37 ("IgG solution"). The preparations produced as controls were subjected to the same process as that for the human IgG-containing gel preparations 11-12 and 11-37.

The results are shown in FIG. 10. The IgG contained (in an included state) in the gel preparation obtained by use of α-cyclodextrin and the gel preparation obtained by use of γ-cyclodextrin remained in the eluates in respective amounts of 106. 1% and 106.6% based on the starting amount. On the other hand, the remaining rate of the human IgG in the eluate of the control was 62.4%. Thus, it was verified that the human IgG contained (in the included state) in the gel preparations had been stabilized against thermal stress.

[Example 12: Behavior of release of protein from gel preparation]

In the same manner as in the preparing method in (1) of Example 2, gel preparations 12-12 and 12-37 of formulation 12 and 37 each containing 115.6 mg/mL of human IgG were each prepared in an amount of 1 g in a test tube. To each of the test tubes was added 5 mL of the solution A, and the test tubes were shaken gently. After the shaking, each of the test tubes containing the gel preparations was gently shaken at 150 rpm at a temperature of 37°C, and the supernatant was sampled with time. The amount of human IgG released was calculated, on the basis of the starting amount of the human IgG contained in the gel preparation, through measurement of the absorbance at 280 nm of the supernatant on a UV spectrophotometer (model V-630, produced by JASCO Corporation). As controls, PEG/cyclodextrin/human IgG mixed solutions were prepared, and subjected to a release test by the same operation as abovementioned. Note that this experiment was conducted with N = 3. The results are set forth in Table 7 below.

[Table 7]

Table 7

| | Amount of IgG released (%) | | | | |
|---|---|---|---|---|---|
| Lapse of time | 1h | 2h | 3h | 6h | 12h |
| Gel preparation 12-12 | 19.2±1.9 | 32.9±0.8 | 46.3±3.8 | 82.6±5.1 | 98.7±4.9 |
| Gel preparation 12-37 | 34.4±2.0 | 51.7±1.6 | 70.3±9.3 | 89.0±5.5 | 97.5±3.8 |
| PEG/α-cyclodextrin / human IgG mixed solution | 95.6±0.8 | 96.2±1.2 | 97.9±1.2 | 102.9±1.3 | 108.0±0.7 |
| PEG/γ-cyclodextrin / human IgG mixed solution | 93.2±0.7 | 96.4±2.1 | 102.4±109 | 104.9±1.5 | 107.9±0.2 |

[Example 13: Inhibitory effect on aggregation induced by agitation stress -4]

Gel preparations 13-42(4), 13-42(8), 13-42(16), and 13-42 (32) of formulation 42 respectively containing 4.0, 8.0, 16.0, and 32.0 mg/mL of human IgG were each prepared in an amount of 250 μL in a microtube, in the same manner as in the preparing method in (1) of Example 2, while using Phosphate buffered saline (PBS, pH 7.4) in place of the solution A. Next, the microtubes were set on a shaking apparatus, and shaking at 500 rpm was conducted at 37°C for about 24 hours, to give an agitation stress to the gel preparations. After the loading with the agitation stress, 0.75 mL of PBS was added to each gel preparation, then the admixture was gently shaken until the gel structure disappeared on a visual observation basis, to disintegrate the gel, and the human IgG was eluted. After each of the human IgG eluates thus obtained was centrifuged to precipitate aggregates, the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 3, and the remaining rate of human IgG was expressed based on the starting amount. Besides, as controls, human IgG solutions prepared by dissolving human IgG in PBS so as to obtain respective concentrations of 4.0, 8.0, 16.0, and 32.0 mg/mL were subjected to the same process as above.

The results are shown in FIG. 11. The remaining rates of the human IgG in the eluates of the controls (IgG solutions) containing 4.0 mg/mL to 32.0 mg/mL of human IgG were 8.7% to 58.7%. On the other hand, the remaining rates of the human IgG in the eluates contained (in an included state) in amounts of 4.0 mg/mL to 32.0 mg/mL in the gel preparations obtained by use of α-cyclodextrin were in the range of 100.0% to 108.2%. Thus, the human IgG contained (in the included state) in the gel preparations had been stabilized against agitation stress.

[Example 14: Inhibitory effect on aggregation induced by agitation stress -5]

Gel preparations 14-52(1), 14-52(4), 14-52(8), and 14-52 (16) of formulation 52 respectively containing 1.0, 4.0, 8.0, and 16.0 mg/mL of human IgG were each prepared in an amount of 250 μL in a microtube, in the same manner as in the preparing method in (1) of Example 2, while using Phosphate buffered saline (PBS, pH 7.4) in place of the solution A. Next, the microtubes were set on a shaking apparatus, and shaking at 37°C and 500 rpm was conducted for five days, to give an agitation stress to each of the gel preparations. After the loading with the agitation stress, 0.75 mL of PBS was added to each gel preparation, then the admixture was gently shaken until the gel structure disappeared on a visual observation basis, thereby disintegrating the gel, and the human IgG was eluted. Each of the IgG eluates obtained was centrifuged to precipitate aggregates, and the absorbance of the supernatant at 280 nm was measured on a UV spectrophotometer (model UV-2450, produced by Shimadzu Corporation). From the absorbance values, the amounts of human IgG contained in the eluates were calculated. Note that this experiment was performed with N = 3, and the remaining rate of human IgG was expressed based on the starting amount. Besides, as controls, human IgG solutions prepared by dissolving human IgG in PBS so as to obtain respective concentrations of 1.0, 4.0, 8.0, and 16.0 mg/mL were subjected to the same process as above.

The results are shown in FIG. 12. The remaining rates of human IgG in the eluates of the controls (IgG solutions) containing 1.0 mg/mL to 16.0 mg/mL of human IgG were 0.9% to 14.3%. On the other hand, the remaining rates of human IgG in the eluates contained (in an included state) in amounts of 1.0 mg/mL to 16.0 mg/mL in the gel preparations produced by use of γ-cyclodextrin were in the range of 95.8% to 107.3%. Thus, the human IgG contained (in the included state) in the gel preparations was found to have been remarkably stabilized against agitation stress.

[Example 15: Inhibitory effect on aggregation induced by agitation stress -6]

A gel preparation 15-41 of formulation 41 containing 240.0 mg/mL of human IgG was prepared in an amount of 250 μL in a microtube, in the same manner as in the preparing method in (1) of Example 2, while using Phosphate buffered saline (PBS, pH 7.4) in place of the solution A. The microtube was shaken under the conditions of 500 rpm and 37°C for five days, to load the gel preparation with an agitation stress. After the loading with the stress, 1 mL of water was added to each sample, followed by gentle shaking to disintegrate the gel preparation. Each sample was centrifuged, and the supernatant was filtered through a membrane filter. In order to determine the amount of human IgG in the eluate, the absorbance of the supernatant at 280 nm was measured on a UV spectrophotometer (model UV-2450, produced by Shimadzu Corporation). From the absorbance value, the remaining rate of the human IgG contained in the eluate was calculated. The remaining rate of the human IgG was expressed based on the starting amount. As a control, a solution prepared by dissolving human IgG in PBS so as to obtain a concentration of 240 mg/mL was subjected to the same process as above. Note that this experiment was conducted with N = 3.

The results are set forth in Table 8 below. The remaining rate of protein in the control was 78.7%, whereas the IgG contained (in an included state) in the gel preparation remained at a rate of 102.5%. Thus, it was verified that the human IgG in the gel preparation had been stabilized against agitation stress.

[Table 8]

Table 8. Remaining rate of human IgG after loaded with agitation stress (%)

| | Gel preparation 15-41 | Human IgG solution |
|---|---|---|
| 1 | 103.1 | 79.4 |
| 2 | 103.7 | 78.6 |
| 3 | 100.7 | 78.1 |
| Average | 102.5 | 78.7 |

[Example 16: Inhibitory effect on aggregation induced by agitation stress -7]

Gel preparations 16-42 to 16-45 of formulations 42 to 45 containing 1.0 mg/mL to 32.0 mg/mL of human IgG were each prepared in an amount of 250 μL in a microtube, in the same manner as in the preparing method in (1) of Example 2, while using Phosphate buffered saline (PBS, pH 7.4) in place of the solution A. Next, the microtubes were set on a shaking apparatus, and shaking at 37°C and 500 rpm was conducted for two days, to give an agitation stress to the gel preparations. After the loading with the agitation stress, 0.75 mL of PBS was added to each gel preparation, then the admixture was shaken gently until the gel structure disappeared on a visual observation basis, thereby disintegrating the gel, and the human IgG was eluted. After each of the IgG eluates was centrifuged to precipitate aggregates, the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 2, and the remaining rate of human IgG was expressed based on the starting amount. Besides, as controls, human IgG solutions prepared by dissolving human IgG in PBS so as to obtain a concentration of 4.0 mg/mL or 8.0 mg/mL were subjected to the same process as above.

The results are set forth in Table 9 below. Note that in regard to Table 9 below and Table 10 described later, the gel preparations which have the same gel concentration but differ in human IgG concentration were given such sample numbers as "16-42-1" and "16-42-2" so that they can be distinguished from each other by the branch number.

The remaining rates of human IgG in the eluates of the controls (human IgG solutions) were 15.5% to 22.2%, and the remaining rates of human IgG contained (in an included state) in the gel preparations 16-45 with a gel concentration of 2.3 wt/vol% obtained by use of α-cyclodextrin were 15.4% to 19.1%. On the other hand, the remaining rates of human IgG contained (in an included state) in the gel preparations 16-44 with a gel concentration of 4.1 wt/vol% were at an improved level of 44.8% to 59.8%. Further, the remaining rate of human IgG contained (in an included state) in the gel preparations 16-42 and 16-43 with a gel concentration of not less than 7.3 wt/vol% were in the range of 99.8% to 102.5%, indicating the stabilization of the human IgG against agitation stress. Thus, it was verified that the gel preparations with a gel concentration of not less than 4 wt/vol% have a stabilizing effect on the human IgG.

[Table 9]

Table 9. Remaining rate of human IgG after loaded with agitation stress (%)

| | Gel concentration (wt/vol%) | Human IgG concentration (mg/mL) | IgG remaining rate (%) |
|---|---|---|---|
| Gel preparation 16-42-1 | 10.9 | 8.0 | 100.5 |
| Gel preparation 16-42-2 | 10.9 | 4.0 | 102.5 |
| Gel preparation 16-43-1 | 7.3 | 32.0 | 100.2 |
| Gel preparation 16-43-2 | 7.3 | 1.0 | 99.8 |
| Gel preparation 16-44-1 | 4.1 | 32.0 | 59.8 |
| Gel preparation 16-44-2 | 4.1 | 8.0 | 44.8 |
| Gel preparation 16-45-1 | 2.3 | 8.0 | 19.1 |
| Gel preparation 16-45-2 | 2.3 | 4.0 | 15.4 |
| Gel preparation 16-45-3 | 2.3 | 1.0 | 19.0 |
| Human IgG solution-1 | - | 8.0 | 22.2 |
| Human IgG solution-2 | - | 4.0 | 15.5 |

[Example 17: Inhibitory effect on aggregation induced by agitation stress -8]

Gel preparations 17-52 and 17-54 to 17-56 of formulations 52 and 54 to 56 containing 1.0 mg/mL to 32.0mg/mL of human IgG were each prepared in an amount of 250 μL in a microtube, in the same manner as in the preparing method in (1) of Example 2, while using Phosphate buffered saline (PBS, pH 7.4) in place of the solution A. Next, the microtubes were set on a shaking apparatus, and shaking at 37°C and 500 rpm was conducted for two days, to give an agitation stress to the gel preparations. After the loading with the agitation stress, 0.75 mL of PBS was added to each gel preparation, then the admixture was gently shaken until the gel structure disappeared on a visual observation basis, thereby disintegrating the gel, and the human IgG was eluted. After each of the IgG eluates thus obtained was centrifuged to precipitate aggregates, the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 2, and the remaining rate of human IgG was expressed based on the starting amount. Besides, as controls, human IgG solutions prepared by dissolving human IgG in PBS so as to obtain a concentration of 1.0 mg/mL or 4.0 mg/mL were subjected to the same process as above.

The results are set forth in Table 10 below. The remaining rates of human IgG in the eluates of the control (IgG solutions) were 8.1% to 8.4%, and the remaining rate of human IgG contained (in an included state) in the gel preparation 17-56 with a gel concentration of 2.2 wt/vol% obtained by use of γ-cyclodextrin was 9.0%. On the other hand, the remaining rates of human IgG contained (in an included state) in the gel preparations 17-55 with a gel concentration of 4.0 wt/vol% were at an improved level of 35.6% to 47.3%. Further, the remaining rates of human IgG contained (in an included state) in the gel preparations 17-52 and 17-54 with a gel concentration of not less than 8.7 wt/vol% were in the range of 92.4% to 100.9%, indicating stabilization of the human IgG against agitation stress. Thus, it was verified that the gel preparations with a gel concentration of not less than 4 wt/vol% have a stabilizing effect on human IgG.

[Table 10]

Table 10. Remaining rate of human IgG after loaded with agitation stress (%)

| | Gel concentration (wt/vol%) | Human IgG concentration (mg/mL) | IgG remaining rate (%) |
|---|---|---|---|
| Gel preparation 17-52-1 | 17.3 | 8.0 | 97.3 |

(continued)

| | Gel concentration (wt/vol%) | Human IgG concentration (mg/mL) | IgG remaining rate (%) |
|---|---|---|---|
| Gel preparation 17-52-2 | 17.3 | 4.0 | 98.8 |
| Gel preparation 17-54-1 | 8.7 | 32.0 | 92.4 |
| Gel preparation 17-54-2 | 8.7 | 1.0 | 100.9 |
| Gel preparation 17-55-1 | 4.0 | 32.0 | 47.3 |
| Gel preparation 17-55-2 | 4.0 | 8.0 | 35.6 |
| Gel preparation 17-56-1 | 2.2 | 8.0 | 9.0 |
| Human IgG solution-1 | - | 4.0 | 8.4 |
| Human IgG solution-2 | - | 1.0 | 8.1 |

[Example 18: Inhibitory effect on aggregation induced by agitation stress -9]

Gel preparations 18-80 and 18-81 of formulations 80 and 81 containing 8.0 mg/mL of human IgG were each prepared in an amount of 250 μL in a microtube, in the same manner as in the preparing method in (1) of Example 2, while using Phosphate buffered saline (PBS, pH 7.4) in place of the solution A and using PEG35000 in place of PEG20000. Next, the microtubes were set on a shaking apparatus, and shaking at 37°C and 500 rpm was conducted for two days, to give an agitation stress to the gel preparations. After the loading with the agitation stress, 0.75 mL of PBS was added to each gel preparation, then the admixture was gently shaken until the gel structure disappeared on a visual observation basis, thereby disintegrating the gel, and the human IgG was eluted. Each of the IgG eluates thus obtained was centrifuged to precipitate aggregates, and thereafter the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 2, and the remaining rate of the human IgG was expressed based on the starting amount. Besides, as controls, IgG solutions prepared by dissolving human IgG in PBS so as to obtain a concentration of 8.0 mg/mL were subjected to the same process as above

The results are set forth in Table 11-1 and Table 11-2 below. The remaining rates of human IgG in the eluates of the controls (IgG solutions) containing 8.0 mg/mL of human IgG were 15.0% and 18.4%. On the other hand, the remaining rates of human IgG contained (in an included state) in the gel preparation 18-80 prepared using α-cyclodextrin and PEG35000 and in the gel preparation 18-81 prepared by use of γ-cyclodextrin and PEG35000 were 98.7% and 96.1%, indicating stabilization of the human IgG against agitation stress.

[Table 11]

Table 11-1. Remaining rate of human IgG after loaded with agitation stress (%)

| | Gel preparation 18-80 | Human IgG solution |
|---|---|---|
| 1 | 98.1 | 15.5 |
| 2 | 99.2 | 14.4 |
| Average | 98.7 | 15.0 |

Table 11-2. Remaining rate of human IgG after loaded with agitation stress (%)

| | Gel preparation 18-81 | Human IgG solution |
|---|---|---|
| 1 | 95.8 | 19.2 |
| 2 | 96.4 | 17.6 |
| Average | 98.1 | 18.4 |

[Example 19: Inhibitory effect on aggregation induced by agitation stress -10]

Gel preparations 19-82 to 19-89 of formulations 82 to 89 containing 8.0 mg/mL of human IgG were each prepared in an amount of 250 μL in a microtube, in the same manner as in the preparing method in (1) of Example 2, while using Phosphate buffered saline (PBS, pH 7.4) in place of the solution A and using PEG20000 and PEG400 in place of PEG20000. Next, the microtubes were set on a shaking apparatus, and shaking at 37°C and 500 rpm was carried out for two days, to give an agitation stress to the gel preparations. After the loading with the agitation stress, 0.75 mL of PBS was added to each gel preparation, then the admixture was shaken gently until the gel structure disappeared on a visual observation basis, thereby disintegrating the gel, and the human IgG was eluted. Each of the human IgG eluates obtained was centrifuged to precipitate aggregates, and therefore the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 2, and the remaining rate of the human IgG was expressed based on the starting amount. Besides, as a control, an IgG solution prepared by dissolving human IgG in PBS so as to obtain a concentration of 8.0 mg/mL was subjected to the same process as above.

The results are set forth in Table 12 below. The remaining rate of human IgG in the eluate of the control (IgG solution) containing 8.0 mg/mL of human IgG was 14.0%. On the other hand, the remaining rates of human IgG contained (in an included state) in the gel preparations 19-82 to 19-89 were in the range of 93.0% to 101.0%, indicating remarkable stabilization of the human IgG against agitation stress.

[Table 12]

Table 12. Remaining rate of human IgG after loaded with agitation stress (%)

| | 1 | 2 | Average |
|---|---|---|---|
| Gel preparation 19-82 | 100.8 | 101.2 | 101.0 |
| Gel preparation 19-83 | 99.4 | 98.4 | 98.9 |
| Gel preparation 19-84 | 95.2 | 95.9 | 95.5 |
| Gel preparation 19-85 | 97.4 | 98.2 | 97.8 |
| Gel preparation 19-86 | 98.0 | 98.4 | 98.2 |
| Gel preparation 19-87 | 98.6 | 97.5 | 98.1 |
| Gel preparation 19-88 | 99.0 | 99.0 | 99.0 |
| Gel preparation 19-89 | 93.4 | 92.7 | 93.0 |
| Human IgG solution | 13.8 | 14.3 | 14.0 |

[Example 20: Inhibitory effect on aggregation induced by agitation stress -11]

Gel preparations 20-90 to 20-93 of formulations 90 to 93 containing 8.0 mg/mL of human IgG were each prepared in an amount of 250 μL in a microtube, in the same manner as in the preparing method in (1) of Example 2, while using Phosphate buffered saline (PBS, pH 7.4) in place of the solution A and using PEG20000 and PEG4000 in place of PEG20000. Next, the microtubes were set on a shaking apparatus, and shaking at 37°C and 500 rpm was conducted for two days, to give an agitation stress to the gel preparations. After the loading with the agitation stress, 0.75 mL of PBS was added to each gel preparation, then the admixture was gently shaken until the gel structure disappeared on a visual observation basis, thereby disintegrating the gel, and the human IgG was eluted. Each of the human IgG eluates obtained was centrifuged to precipitate aggregates, and then the absorbance of the supernatant at 280 nm was measured on a UV spectrophotometer (model V-650, produced by JASCO Corporation). From the absorbance values, the amounts of human IgG contained in the eluates were calculated. Note that this experiment was conducted with N = 2, and the remaining rate of human IgG was expressed based on the starting amount. Besides, as a control, a human IgG solution prepared by dissolving human IgG in PBS so as to obtain a concentration of 8.0 mg/mL was subjected to the same process as above.

The results are set forth in Table 13 below. The remaining rate of human IgG in the eluate of the control (human IgG solution) containing 8.0 mg/mL of human IgG was 14.0%. On the other hand, the remaining rates of human IgG contained (in an included state) in the gel preparations 20-90 to 20-93 were in the range of 97.5% to 104.4%, indicating remarkable stabilization of the human IgG against agitation stress.

[Table 13]

Table 13. Remaining rate of human IgG after loaded with agitation stress (%)

| | 1 | 2 | Average |
|---|---|---|---|
| Gel preparation 20-90 | 102.1 | 99.8 | 101.0 |
| Gel preparation 20-91 | 97.5 | 97.4 | 97.5 |
| Gel preparation 20-92 | 102.7 | 106.1 | 104.4 |
| Gel preparation 20-93 | 101.6 | 100.4 | 101.0 |
| Human IgG solution | 13.8 | 14.3 | 14.0 |

[Example 21: Inhibitory effect on aggregation induced by agitation stress -12]

A gel preparation 21-46 of formulation 46 containing 8.0 mg/mL of human IgG was prepared in an amount of 250 μL in a microtube, in the same manner as in the preparing method in (1) of Example 2, while using Phosphate buffered saline (PBS, pH 7.4) in place of the solution A. Next, the microtube was set on a shaking apparatus, and shaking at 37°C and 500 rpm was conducted for two days, to give an agitation stress to the gel preparation. After the loading with the agitation stress, 0.75 mL of PBS was added to the gel preparation, then the admixture was shaken gently until the gel structure disappeared on a visual observation basis, thereby disintegrating the gel, and the human IgG was eluted. The IgG eluate obtained was centrifuged to precipitate aggregates, and thereafter the absorbance of the supernatant at 280 nm was measured on a UV spectrophotometer (model V-650, produced by JASCO Corporation) . From the absorbance value, the amount of human IgG contained in the eluate was calculated. Note that this experiment was conducted with N = 2, and the remaining rate of human IgG was expressed based on the starting amount. Besides, as a control, a human IgG solution prepared by dissolving human IgG in PBS so as to obtain a concentration of 8.0 mg/mL was subjected to the same process as above.

The results are set forth in Table 14 below. The remaining rate of human IgG in the eluate of the control (human IgG solution) containing 8.0 mg/mL of human IgG was 14.0%. On the other hand, the remaining rate of human IgG contained (in an included state) in the gel preparation 21-46 was 99.2%, showing remarkable stabilization of the human IgG against agitation stress.

[Table 14]

Table 14. Remaining rate of human IgG after loaded with agitation stress (%)

| | Gel preparation 21-46 | Human IgG solution |
|---|---|---|
| 1 | 98.8 | 13.7 |
| 2 | 99.6 | 14.3 |
| Average | 99.2 | 14.0 |

[Example 22: Inhibitory effect on aggregation induced by agitation stress -13]

Gel preparations 22-47 and 22-57 of formulations 47 and 57 containing 4.0 mg/mL of anti-RS virus antibody were each prepared in an amount of 250 μL in a microtube, in the same manner as in the preparing method in (1) of Example 2, while using a solution B containing 3.9 mg/mL of L-histidine and 0.12 mg/mL of glycine, in place of the solution A. The microtubes were shaken under the conditions of 37°C and 500 rpm for five days, to give an agitation stress to the gel preparations. After the loading with the stress, 750 μL of the solution B was added to each sample, and the resulting admixture was shaken gently to disintegrate the gel preparation. Each of the samples was centrifuged, and the supernatant was filtered through a membrane filter. In order to analyze the anti-RS virus antibody in each eluate, the absorbance of the supernatant at 280 nm was measured on a UV spectrophotometer (model: V-650, produced by JASCO Corporation). From the absorbance values obtained, the remaining rates of the anti-RS virus antibody contained in the eluates were calculated. The remaining rate of anti-RS virus antibody was expressed based on the starting amount. As a control, an anti-RS virus antibody solution prepared by dissolving the anti-RS virus antibody in water so as to obtain a concentration of 4.0 mg/mL was subjected to the same process as above. Note that this experiment was conducted with N = 3.

The results are set forth in Table 15 below. The remaining rate of the protein in the control was 43.1%. On the other hand, the anti-RS virus antibody contained (in an included state) in the gel preparations remained at rates of 111.0% and 110.5%, indicating that the anti-RS virus antibody had been stabilized against agitation stress.

[Table 15]

Table 15. Remaining rate of anti-RS virus antibody after loaded with agitation stress (%)

| | Gel preparation 22-47 | Gel preparation 22-57 | Anti-RS virus antibody solution |
|---|---|---|---|
| 1 | 112.0 | 110.3 | 73.4 |
| 2 | 110.8 | 109.6 | 39.9 |
| 3 | 110.3 | 111.7 | 15.9 |
| Average | 111.0 | 110.5 | 43.1 |

[Example 23: Inhibitory effect on aggregation induced by agitation stress -14]

Gel preparations 23-40 and 23-50 of formulations 40 and 50 containing 4.0 mg/mL of anti-epidermal growth factor receptor (EGFR) antibody were each prepared in an amount of 200 μL in a microtube, in the same manner as in the preparing method in (1) of Example 2, while using a solution C (pH 6.0) containing 6.8 mg/mL of sodium acetate hydrate and 5.8 mg/mL of sodium chloride, in place of the solution A. The microtubes were shaken under the conditions of 37°C and 500 rpm for five days, thereby loading the gel preparations with an agitation stress. After the loading with the stress, 800 μL of the solution C was added to each sample, and the resulting admixture was shaken gently to disintegrate the gel preparation. Each of the samples were then centrifuged, and the supernatant was filtered through a membrane filter. In order to analyze the anti-EGFR antibody in each eluate, the absorbance of the supernatant at 280 nm was measured on a UV spectrophotometer (model V-650, produced by JASCO Corporation). From the absorbance values obtained, the remaining rates of anti-EGFR antibody contained in the eluates were calculated. The remaining rate was expressed based on the starting amount. As a control, an anti-EGFR antibody solution prepared by dissolving the anti-EGFR antibody in the solution C so as to obtain a concentration of 4.0 mg/mL was subjected to the same process as above. Note that this experiment was conducted with N = 3.

The results are set forth in Table 16 below. The remaining rate of the anti-EGFR antibody in the control was 14.5%. On the other hand, the anti-EGFR antibody contained (in an included state) in the gel preparations remained in rates of 106.1% and 107.4%, indicating remarkable stabilization of the anti-EGFR antibody against agitation stress.

[Table 16]

Table 16. Remaining rate of anti-EGFR antibody after loaded with agitation stress (%)

| | Gel preparation 23-40 | Gel preparation 23-50 | Anti-EGFR antibody solution |
|---|---|---|---|
| 1 | 105.9 | 106.7 | 4.8 |
| 2 | 105.3 | 105.8 | 13.3 |
| 3 | 107.1 | 109.7 | 25.4 |
| Average | 106.1 | 107.4 | 14.5 |

[Example 24: Inhibitory effect on aggregation induced by agitation stress -15]

Gel preparations 24-40 and 24-50 of formulations 40 and 50 containing 10.0 mg/mL of TNFα/LTα receptor fusion protein were each prepared in an amount of 250 μL in a 2. 5-mL disposable syringe (produced by Terumo Corporation), in the same manner as in the preparing method in (1) of Example 2 while using the solution A. The syringes were shaken under the conditions of 500 rpm and 37°C for five days, to load the gel preparations with an agitation stress. After the loading with the stress, 250 μL of the solution A was added to each sample, and the resulting admixture was shaken gently, to disintegrate the gel preparation. Each sample was then centrifuged, the supernatant was filtered through a membrane filter, and the TNFα/LTα receptor fusion protein in each eluate was subjected to a size-based analysis by size exclusion chromatography. As controls, there were prepared a preparation obtainable by removing cyclodextrin from the gel preparation 24-50 containing 10.0 mg/mL of the TNFα/LTα receptor fusion protein ("TNFα/LTα receptor + PEG"), preparations each obtainable by removing PEG20000 from the gel preparations 24-40 and 24-50 ("TNFα/LTα receptor + α-CyD" and "TNFα/LTα receptor + γ-CyD"), and a preparation obtainable by removing cyclodextrin and PEG20000 from the gel preparation 24-50 ("TNFα/LTα receptor solution"), and they were subjected to the same process as above. As the remaining rate, the area of a monomer peak for the stressed sample was expressed in %, based on the area of a monomer peak for a TNFα/LTα receptor fusion protein solution on which the agitation stress had not been exerted. In addition, the area of soluble aggregate peaks was expressed in ratio (%) to the total area of all peaks. Note that the soluble aggregate peaks were peaks assumed to correspond to a molecular weight of not less than 670,000 from an analysis using a molecular weight marker. This experiment was conducted with N = 3.

The results are set forth in FIG. 13A and FIG. 13B. The remaining rates of the TNFα/LTα receptor fusion protein in the solutions of the controls were not more than 94%. On the other hand, the TNFα/LTα receptor fusion protein contained (in an included state) in the gel preparations remained at rates of 104.9% and 99.2% (FIG. 13A). Besides, aggregate peaks in a ratio of 1.3% to 8.7% to the total area were observed for the solutions of the controls, whereas such peaks were not observed for the gel preparations. This shows that the TNFα/LTα receptor fusion protein in the gel preparations had been stabilized against agitation stress (FIG. 13B).

[Example 25: Inhibitory effect on aggregation induced by agitation stress -16]

Gel preparations 25-40 and 25-50 of formulations 40 and 50 respectively containing 3.8 mg/mL and 7.5 mg/mL of L-asparaginase were each prepared in an amount of 250 μL in a microtube, in the same manner as in the preparing method in (1) of Example 2, while using water in place of the solution A. The microtubes were shaken under the conditions of 500 rpm and 37°C for seven days, to load the gel preparations with an agitation stress. After the loading with the stress, 800 μL of water was added to each sample, and the resulting admixture was shaken gently to disintegrate the gel preparation. Each sample was centrifuged, the supernatant was filtered through a membrane filter, and L-asparaginase in the eluate was subjected to a size-based analysis by size exclusion chromatography. As controls, L-asparaginase solutions prepared by dissolving L-asparaginase in water so as to obtain a concentration of 3.8 mg/mL or 7.5 mg/mL were subjected to the same process as above. As the remaining rate, the areas of monomer peaks for the stressed samples were expressed in % based on the area of a monomer peak for the L-asparaginase to which the agitation stress had not been applied. Note that this experiment was conducted with N = 3.

The results are set forth in Table 17-1 and Table 17-2 below. The remaining rate of L-asparaginase in the control (3.8 mg/mL) was 11.7%, whereas L-asparaginase in the gel preparations containing L-asparaginase (3.8 mg/mL) remained at rates of 100.9% and 104.1% (Table 17-1). Besides, the remaining rate of L-asparaginase in the control (7.5 mg/mL) was 23.4%, whereas L-asparaginase in the gel preparations containing L-asparaginase (7.5 mg/mL) remained at rates of 101.2% and 105.8% (Table 17-2). Thus, the L-asparaginase in the gel preparations had been remarkably stabilized against agitation stress.

[Table 17]

Table 17-1. Remaining rate of L-asparaginase (3.8 mg/mL) monomer peak after loaded with agitation stress (%)

| | Gel preparation 25-40 | Gel preparation 25-50 | L-asparaginase solution |
|---|---|---|---|
| 1 | 102.4 | 104.3 | 10.6 |
| 2 | 99.9 | 102.5 | 9.8 |
| 3 | 100.3 | 105.6 | 14.6 |

(continued)

| | Gel preparation 25-40 | Gel preparation 25-50 | L-asparaginase solution |
|---|---|---|---|
| Average | 100.9 | 104.1 | 11.7 |

Table 17-2. Remaining rate of L-asparaginase (7.5 mg/mL) monomer peak after loaded with agitation stress (%)

| | Gel preparation 25-40 | Gel preparation 25-50 | L-asparaginase solution |
|---|---|---|---|
| 1 | 102.2 | 106.5 | 14.4 |
| 2 | 101.0 | 101.8 | 26.9 |
| 3 | 100.4 | 109.0 | 28.9 |
| Average | 101.2 | 105.8 | 23.4 |

[Example 26: Inhibitory effect on aggregation induced by agitation stress -17]

Gel preparations 26-40 and 26-50 of formulations 40 and 50 containing 1.0 mg/mL of anti-VEGF-A antibody Fab fragment were each prepared in an amount of 250 μL in a microtube, in the same manner as in the preparing method in (1) of Example 2, while using water in place of the solution A. The microtubes were shaken under the conditions of 500 rpm and 37°C for two days, to load the gel preparations with an agitation stress. After the loading with the stress, 250 μL of water was added to each sample, and the resulting admixture was shaken gently, to disintegrate the gel preparation. Each of the samples was centrifuged, the supernatant was filtered through a membrane filter, and the anti-VEGF-A antibody Fab fragment in the eluate was subjected to a size-based analysis by size exclusion chromatography. As a control, an anti-VEGF-A antibody Fab fragment solution prepared by dissolving anti-VEGF-A antibody Fab fragment in water so as to obtain a concentration of 1.0 mg/mL was subjected to the same process as above. As the remaining rate, the areas of monomer peaks for the stressed samples were expressed in % based on the area of a monomer peak for the anti-VEGF-A antibody Fab fragment solution to which the agitation stress had not been applied. Note that this experiment was conducted with N = 3.

The results are set forth in Table 18 below. The monomer remaining rate for the anti-VEGF-A antibody Fab fragment in the control was 8.8%. On the other hand, the anti-VEGF-A antibody Fab fragment contained (in an included state) in the gel preparations remained at rates of 100.6% and 102.4%. Thus, the anti-VEGF-A antibody Fab fragment in the gel preparations was found to have been remarkably stabilized against agitation stress.

[Table 18]

Table 18. Remaining rate of anti-VEGF-A antibody monomer peak after loaded with agitation stress (%)

| | Gel preparation 26-40 | Gel preparation 26-50 | Anti-VEGF-A antibody Fab fragment solution |
|---|---|---|---|
| 1 | 100.1 | 101.5 | 8.1 |
| 2 | 101.0 | 103.5 | 9.0 |
| 3 | 100.0 | 102.2 | 9.2 |
| Average | 100.6 | 102.4 | 8.8 |

[Example 27: Inhibitory effect on aggregation induced by agitation stress -18]

Gel preparations 27-41 and 27-51 of formulations 41 and 51 containing 7.0 mg/mL of antihuman IgE antibody were each prepared in an amount of 250 μL in a microtube, in the same manner as in the preparing method in (1) of Example 2, while using water in place of the solution A. The microtubes were shaken under the conditions of 500 rpm and 37°C for five days, to load the gel preparations with an agitation stress. After the loading with the stress, each of the gel preparations was disintegrated by a microspatula and centrifuged. Thereafter, the supernatant was filtered through a membrane filter, and the antihuman IgE antibody in each supernatant was subjected to the following evaluation. Note that this experiment was conducted with N = 2.

I. The absorbance of each supernatant at 280 nm was measured on a UV spectrophotometer (model V-650, produced by JASCO Corporation). From the absorbance values, the remaining rates of antihuman IgE antibody in the supernatants were calculated. As a control, an antihuman IgE antibody solution prepared by dissolving antihuman IgE antibody in water so as to obtain a concentration of 7. 0 mg/mL was subjected to the same process as above, exclusive of the disintegrating operation by the microspatula. The remaining rate of antihuman IgE antibody was expressed based on the starting amount.

II. Protein concentrations were calibrated based on the results of I above. Thereafter, as antihuman IgE antibody activity, a human IgE binding inhibition activity of the antihuman IgE antibody in the supernatant was measured by an ELISA method using a plate on which FcεR1 receptor had been provided in a solid phase. Activity retention rate was calculated (in %) based on an antihuman IgE antibody solution which had not been loaded with the agitation stress.

The results are set forth in Table 19-1 and Table 19-2 below.

I. The remaining rate of antihuman IgE antibody in the control (7.0-mg/mL antihuman IgE antibody solution) was 76.5%, whereas the antihuman IgE antibody contained (in an included state) in the gel preparations remained at rates of 97.1% and 108.2% (Table 19-1).

II. The antihuman IgE antibody contained (in an included state) in the gel preparations retained the activity at rates of 90.6% and 90.3% even after loaded with the agitation stress (Table 19-2).

From the above results, it was made clear that the antihuman IgE antibody contained (in the included state) in the gel preparations had been stabilized against agitation stress.

[Table 19]

Table 19-1. Remaining rate of antihuman IgE antibody after loaded with agitation stress (%)

| | Gel preparation 27-41 | Gel preparation 27-51 | Antihuman IgE antibody solution |
|---|---|---|---|
| 1 | 100.0 | 107.7 | 76.1 |
| 2 | 94.2 | 108.6 | 76.8 |
| Average | 97.1 | 108.2 | 76.5 |

Table 19-2. Activity retention rate of antihuman IgE antibody in gel preparation (%)

| | Gel preparation 27-41 | Gel preparation 27-51 |
|---|---|---|
| 1 | 86.3 | 89.6 |
| 2 | 94.8 | 90.9 |
| Average | 90.6 | 90.3 |

[Example 28: Inhibitory effect on aggregation induced by agitation stress -19]

A gel preparation 28-48 of formulation 48 containing 0.1 mg/mL of α-human atrial natriuretic polypeptide (hereinafter referred also to as α-hANP) was prepared in an amount of 500 μL in a microtube, in the same manner as in the preparing method in (1) of Example 2, while using a solution D (pH 4.8) containing 37.5 mg/mL of D-mannitol, 5 mg/mL of glycine, and 9 mg/mL of a sodium chloride solution, in place of the solution A. Next, the microtube was set on a shaking apparatus, and shaking at 37°C and 500 rpm was conducted for five days, to give an agitation stress to the gel preparation. After the loading with the agitation stress, 500 μL of water was added to the sample, and the gel preparation was disintegrated by a microspatula and centrifuged. The supernatant was filtered through a membrane filter, to remove aggregates, and the amount of α-hANP contained in the solution was determined by an HPLC method. Note that this experiment was conducted with N = 3, and the remaining rate of α-hANP was expressed based on the starting amount.

Besides, as a control, an α-hANP solution prepared by dissolving α-hANP in the solution D so as to obtain a concentration of 0.1 mg/mL was subjected to the same process as above, exclusive of the disintegrating operation by the microspatula.

The results are set forth in Table 20 below. The remaining rate of α-hANP in the control (α-hANP solution) was 31.9%, whereas the α-hANP contained (in an included state) in the gel preparation remained at a rate of 90.8%. Thus, the α-hANP contained (in the included state) in the gel preparation had been stabilized against agitation stress.

[Table 20]

Table 20. Remaining rate of α-hANP after loaded with agitation stress (%)

| | Gel preparation 28-48 | α-hANP solution |
|---|---|---|
| 1 | 90.5 | 28.9 |
| 2 | 92.1 | 30.5 |
| 3 | 89.9 | 36.4 |
| Average | 90.8 | 31.9 |

[Example 29: Aggregation-inhibiting effect against oxidative stress -1]

A gel preparation 29-48 of formulation 48 containing 0.1 mg/mL of α-hANP was prepared in an amount of 500 μL in a microtube, in the same manner as in the preparing method in (1) of Example 2, while using a solution E (pH 4.8) containing 37.5 mg/mL of D-mannitol and 5 mg/mL of glycine, in place of the solution A. Then, 50 μL of 0.2% hydrogen peroxide was added to the gel preparation, followed by incubation at 37°C for one hour. Immediately after the incubation, 200 μL of 0.4 M methionine and 250 μL of water were added to the sample, and the gel preparation was disintegrated by a microspatula. The sample was then centrifuged, the supernatant was filtered through a membrane filter to remove aggregates, and the amount of α-hANP contained in the solution was determined by the HPLC method. Note that this experiment was conducted with N = 2, and the remaining rate of α-hANP was expressed based on the starting amount. Besides, as a control, an α-hANP solution prepared by dissolving α-hANP in the solution E so as to obtain a concentration of 0.1 mg/mL was subjected to the same process as above, exclusive of the disintegrating operation by the microspatula.

The results are set forth in Table 21 below. The remaining rate of α-hANP in the control (α-hANP solution) was 65.1%. On the other hand, the α-hANP contained (in an included state) in the gel preparation remained at a rate of 78.3%. This verifies an inhibitory effect on the denaturation of the α-hANP contained (in the included state) in the gel preparation, which would be caused by oxidative stress, and on the aggregation which would result from such denaturation.

[Table 21]

Table 21. Remaining rate of α-hANP after loaded with oxidative stress (%)

| | Gel preparation 29-48 | α-hANP solution |
|---|---|---|
| 1 | 78.8 | 65.4 |
| 2 | 77.8 | 64.7 |
| Average | 78.3 | 65.1 |

[Example 30: Aggregation-inhibiting effect against oxidative stress -2]

A gel preparation 30-41 of formulation 41 containing 1.1 mg/mL of anti-TNFα antibody was prepared in an amount of 900 μL in a microtube, in the same manner as in the preparing method in (1) of Example 2, while using water in place of the solution A. Then, 100 μL of 0.1% hydrogen peroxide was added to the sample, followed by incubation at 37°C for one hour. Immediately after the incubation, 200 μL of 0.4 M methionine was added to the sample, and the gel preparation was disintegrated by a microspatula. The sample was then centrifuged, and the supernatant was filtered through a membrane filter. After reduction and alkylation of the filtered supernatant, peptide mapping (HPLC method) by trypsin digestion was conducted, to determine the peak area that is reduced by oxidation. Besides, as a control, an anti-TNFα antibody solution prepared by dissolving anti-TNFα antibody in water so as to obtain a concentration of 1.1 mg/mL was subjected to the same process as above, exclusive of the disintegrating operation by the microspatula, to determine the peak area that is reduced by oxidation. Note that this experiment was conducted with N = 2.

A comparison between the peak areas which are reduced by oxidation revealed that the peak area for the gel preparation was about 20% larger than the peak area for the control; specifically, the ratio of the peak area for the gel preparation to the peak area for the control was 1.21. This verified an inhibitory effect on the denaturation of the anti-TNFα antibody contained (in an included state) in the gel preparation, which would be caused by oxidative stress, and on the aggregation which would result from such denaturation.

[Example 31: Inhibitory effect on aggregation induced by tungsten]

It is known that tungsten, which may mix into a prefilled syringe or the like, can cause aggregation of protein. Taking this into consideration, the following experiment was carried out, in order to examine whether the gel preparation of the present invention has an inhibitory effect on tungsten-induced aggregation.

A gel preparation 31-58 of formulation 58 containing 1.0 mg/mL of human IgG was prepared in an amount of 900 μL in a microtube, in the same manner as in the preparing method in (1) of Example 2, while using acetate buffer (pH 5.0) in place of the solution A. To this microtube, 100 μL of a 500 ppm sodium tungstate (VI) solution (pH 5.0, acetate buffer) was added, and the sample was left to stand overnight at 4°C, to load the gel preparation with a stress due to tungsten. After the loading with the stress, the gel preparation was disintegrated by a microspatula and was centrifuged. Thereafter, the supernatant was filtered through a membrane filter to remove aggregates, and the absorbance of the filtered supernatant at 280 nm was measured on a UV spectrophotometer (model UV-2450, Shimadzu Corporation). From the absorbance value, the amount of human IgG contained in the eluate was calculated. Note that this experiment was conducted with N = 3, and the remaining rate of the human IgG was expressed based on the starting amount. Besides, as a control, a human IgG solution prepared by dissolving human IgG in the acetate buffer (pH 5.0) so as to obtain a concentration of 1.0 mg/mL was subjected to the same process as above, exclusive of the disintegrating operation by the microspatula.

The results are set forth in Table 22 below. The remaining rate of human IgG in the control (IgG solution) was 78.6%. On the other hand, the IgG contained (in an included state) in the gel preparation remained at a rate of 90.0%. Thus, it was made clear that the human IgG contained (in the included state) in the gel preparation had been effectively stabilized against tungsten-induced aggregation.

[Table 22]

Table 22. Remaining rate of human IgG after addition of tungsten (%)

| | Gel preparation 31-58 | Human IgG solution |
|---|---|---|
| 1 | 92.8 | 78.5 |
| 2 | 91.7 | 79.4 |
| 3 | 85.6 | 78.0 |
| Average | 90.0 | 78.6 |

[Example 32: Inhibitory effect on denaturation induced by agitation stress]

Gel preparations 32-49 and 32-59 of formulations 49 and 59 containing 4.0 mg/mL of anti-epidermal growth factor receptor (EGFR) antibody were each prepared in an amount of 250 μL in a microtube, in the same manner as in the preparing method in (1) of Example 2, while using Phosphate buffered saline (PBS, pH 7. 4) in place of the solution A. The microtubes were shaken under the conditions of 500 rpm and 37°C for five days, thereby loading the gel preparation with an agitation stress. After the loading with the stress, 30 mL of PBS was added to the gel preparation, then the resulting admixture was shaken gently until the gel structure disappeared on a visual observation basis, to disintegrate the gel, and the anti-EGFR antibody was eluted. After the anti-EGFR antibody solution was centrifuged to precipitate aggregates, the absorbance of the supernatant at 280 nm was measured on a UV spectrophotometer (model V-630, produced by JASCO Corporation). From the absorbance value, the concentration of the anti-EGFR antibody contained in the eluate was calibrated, and circular dichroism spectrum was measured by use of a circular dichroism spectrometer (model J-820, produced by JASCO Corporation). As a comparative example, a preparation obtainable by removing cyclodextrin and PEG20000 from the gel preparation 32-59 ("anti-EGFR antibody solution") was prepared and subjected to the same process as above. In addition, an anti-EGFR antibody solution which had not been loaded with the agitation stress was subjected to measurement of circular dichroism spectrum, for use as a control.

The results are shown in FIG. 14. The CD spectrum of the anti-EGFR antibody solution loaded with the agitation stress as the comparative example was characterized in that a peak around 200 nm was increased in the positive direction and a peak around 216 nm was increased in the negative direction, as compared with the CD spectrum of the control. On the other hand, the gel preparations 32-49 and 32-59 were showed suppressed variations, and were stabilized against agitation stress in regard to secondary structure, as well.

[Example 33: Measurement of gel concentration (Poloxamer formulation is not according to the invention)

Gels 33-94 to 33-103 of formulations 94 to 103 were prepared according to compositions set forth in Table 23 below, in the same manner as in Example 1, except for using various polyoxyethylene-polyoxypropylene-polyoxyethylene block copolymers (PEG-PPG-PEG; poloxamers) in place of PEG20000. The blank columns in Table 23 below mean that the relevant components were not contained.

Note that poloxamers used were poloxamer 124 (product name: Lutrol (registered trademark) L 44), poloxamer 188 (product name: Lutrol (registered trademark) F 68), poloxamer 237 (product name: Lutrol (registered trademark) F 87), poloxamer 338 (product name: Lutrol (registered trademark) F 108), and poloxamer 407 (product name: Lutrol (registered trademark) F 127), all produced by BASF Corporation. Besides, α-cyclodextrin bearing a product name Celldex (registered trademark) A-100 (produced by Nihon Shokuhin Kako Co., Ltd.) was used as the α-cyclodextrin, and γ-cyclodextrin bearing a product name DexyPearl γ-100 (produced by Ensuiko Sugar Refining Co., Ltd.) was used as the γ-cyclodextrin.

Next, the gel concentrations (wt/vol%) of the above-mentioned gels 33-94 to 33-103 were measured according to the method described in Example 1. The gel concentrations are set forth in Table 23 below.

[Table 23]

Table 23

| Formulation No. | 94 | 95 | 96 | 97 | 98 |
|---|---|---|---|---|---|
| α-Cyclodextrin (mg/mL) | 112 | 124.9 | 122.3 | 125.4 | 122.3 |
| Poloxamer 124(mg/mL) | 22.7 | | | | |
| Poloxamer 188(mg/mL) | | 13.9 | | | |
| Poloxamer 237(mg/mL) | | | 15.6 | | |

| | | | | | |
|---|---|---|---|---|---|
| Poloxamer 338(mg/mL) | | | | 13.5 | |
| Poloxamer 407(mg/mL) | | | | | 15.6 |
| Gel concentration (wt/vol%) | 13.4 | 13.8 | 14.2 | 13.7 | 13.8 |

| Formulation No. | 99 | 100 | 101 | 102 | 103 |
|---|---|---|---|---|---|
| γ-Cyclodextrin (mg/mL) | 116.0 | 103.1 | 116.0 | 103.1 | 116.0 |
| Poloxamer 124(mg/mL) | 50.0 | | | | |
| Poloxamer 188(mg/mL) | | 55.6 | | | |
| Poloxamer 237(mg/mL) | | | 50.0 | | |
| Poloxamer 338(mg/mL) | | | | 55.6 | |
| Poloxamer 407(mg/mL) | | | | | 50.0 |
| Gel concentration (wt/vol%) | 16.2 | 16.9 | 17.5 | 16.4 | 17.2 |

As shown in the results set forth in Table 23 above, while using five kinds of poloxamers, gels having a gel concentration of not less than 4 wt/vol% could be obtained.

[Example 34: Analysis of crystal structure of gel preparations (Poloxamer formulation is not according to the invention)

(1) Preparation of gel preparation

Gel preparations 34-97, 34-98, 34-102 and 34-103 of formulations 97, 98, 102 and 103 in Table 23 of Example 33 containing 100 mg/mL of human IgG were prepared according to the same procedure as in Example 1, while using various polyoxyethylene-polyoxypropylene-polyoxyethylene block copolymers (PEG-PPG-PEG; poloxamers) in place of PEG20000.

(2) Powder X-ray diffraction measurement

The gel preparations produced in (1) were washed with 100 μL of water, and dried under reduced pressure at room temperature (25°C) for at least three days. Powder X-ray diffraction measurement was conducted for (e) solid samples 34-97a and 34-102a of human IgG-containing gel preparations 34-97 and 34-102, and (j) solid samples 34-98a and 34-103a of human IgG-containing gel preparations 34-98 and 34-103. Also, powder X-ray diffraction measurement was conducted for (a) and (f) cyclodextrin alone, (b) poloxamer 338 alone, (g) poloxamer 407 alone, (c) and (h) human IgG alone, (d) a poloxamer 338/cyclodextrin/human IgG mixture, and (i) a poloxamer 407/cyclodextrin/human IgG mixture, as comparative solid samples.

Note that the comparative solid samples (d) and (i) were obtained by mixing the respective solids in predetermined mixing ratios (respective mixing ratios of 97 and 102, and 98 and 103). FIG. 15A, FIG. 15B, FIG. 15C, and FIG. 15D show the results of powder X-ray diffraction for the gel preparation solid samples 34-97a, 34-98a, 34-102a, and 34-103a.

In the α-cyclodextrin system, the physical mixtures with poloxamer showed a diffraction pattern of a cage structure, like α-cyclodextrin alone. On the other hand, the solid samples 34-97a and 34-98a of the human IgG-containing gel preparations 34-97 and 34-98 gave characteristic peaks in the vicinity of 2θ = 7.5 degrees, 13 degrees, 20 degrees, and 22 degrees, thereby showing a diffraction pattern of a hexagonal system tubular structure, which is similar to the crystal structure of the solid sample 2-12a of the gel preparation 2-12 produced using α-cyclodextrin and PEG in Example 2. In the γ-cyclodextrin system, also, γ-cyclodextrin alone and the physical mixtures with poloxamer showed a diffraction pattern of a cage structure. On the other hand, the solid sample 34-103a of the human IgG-containing gel preparation 34-103 gave a characteristic peak of tetragonal system tubular structure in the vicinity of 2θ = 7.5 degrees, and the solid sample 34-102a of the gel preparation 34-102 gave characteristic peaks of tetragonal system tubular structure in the vicinity of 2θ = 7.5 degrees and 21 degrees, showing that their crystal structures are similar to the crystal structure of the solid sample 2-37a of the gel preparation 2-37 produced using γ-cyclodextrin and PEG in Example 2.

[Example 35: Inhibitory effect on aggregation induced by thermal stress (Poloxamer formulation)]

Gel preparations 35-97 and 35-102 of formulations 97 and 102 containing 100 mg/mL of human IgG were each prepared in an amount of 0.5 mL in a microtube, in the same manner as in the preparing method in Example 33, while using water in place of the solution A and using polyoxyethylene-polyoxypropylene-polyoxyethylene block copolymer (PEG-PPG-PEG; poloxamer 338) in place of PEG20000. The gel preparation in an amount of 0.5 mL was heated on a hot bath at 60°C for 15 minutes, thereby applying a thermal stress to the gel preparation. After the loading with the thermal stress, 30 mL of the solution A was added to the sample, and the admixture was shaken gently until the gel structure disappeared on a visual observation basis, thereby disintegrating the gel, and the human IgG was eluted. After the human IgG eluate was centrifuged to precipitate aggregates, the absorbance of the supernatant at 280 nm was measured on a UV spectrophotometer (model V-630, produced by JASCO Corporation). From the absorbance value, the amount of the human IgG contained in the supernatant was calculated. Note that this experiment was conducted with N = 3, and the remaining rate of human IgG was expressed based on the starting amount.

Besides, as a control, an "IgG solution" containing 100 mg/mL of human IgG was prepared, and was subjected to the same process as that for the human IgG-containing gel preparations 35-97 and 35-102.

The results are shown in FIG. 16. The human IgG contained (in an included state) in the gel preparation 35-97 prepared using α-cyclodextrin and the gel preparation 35-102 prepared using γ-cyclodextrin remained in the eluate respectively in amounts of 100.6% and 92.6% based on their starting amount. On the other hand, the remaining rate of human IgG in the eluate of the control was 73.2%. This clearly shows the stability against thermal stress of the human IgG that was contained (in the included state) in the gel preparation.

As shown in the aforementioned Examples, it can be understood that the gel preparation of the present invention contains the protein in an included state, protects the protein against a physical or chemical stress, inhibits the protein from aggregation, and thereby permits the protein to exist stably.

## Claims

1. A gel preparation which is thixotropic and in which an included protein is protected from a physical or chemical stress, is inhibited from aggregation, and is thereby let exist stably, wherein the gel preparation contains a protein, a diluent, one or more kinds of cyclic molecules, and one or more polyethylene glycols contained in the gel preparation in a concentration of 0.5 mg/mL to 200 mg/mL,
   the protein is contained in the gel preparation in a concentration of 2.5 mg/mL to 400 mg/mL, and
   a gel concentration represented by the following formula [1] is 7 wt/vol% to 30 wt/vol%:
   [Mathematical formula 1]

$$\text{Gel concentration } (wt/vol\%) = \frac{Solids\ weight\ of\ gel\ (g)}{Volume\ of\ gel\ (mL)} \times 100 \qquad [1]$$

   Solids weight of gel (g): Weight of precipitate (solids) obtained by centrifugation of gel (g), wherein the cyclic molecule is cyclodextrin or a derivative thereof.

2. The gel preparation according to claim 1, wherein the physical stress is at least one of heat and agitation.

3. The gel preparation according to claim 1, wherein the chemical stress is at least one of pH, oxidation, and a metal-catalyzed reaction.

4. The gel preparation according to any one of claims 1 to 3, wherein the cyclodextrine is selected from α-cyclodextrin, β-cyclodextrin and γ-cyclodextrin, and the derivative is selected from a methylated cyclodextrin, a maltosylated cyclodextrin, a hydroxypropylated cyclodextrin, a glucosylated cyclodextrin and a sulfobutylated cyclodextrin.

5. The gel preparation according to any one of claims 1 to 4, wherein the protein is a protein for medical use.

6. A method of stabilizing a protein, comprising
including the protein in a gel preparation which contains one or more kinds of cyclic molecules and one or more polyethylene glycols contained in the gel preparation in a concentration of 0.5 mg/mL to 200 mg/mL,

the protein is contained in the gel preparation in a concentration of 2.5 mg/mL to 400 mg/mL, and
a gel concentration represented by the following formula [1] is 7 wt/vol% to 30 wt/vol%:
[Mathematical formula 1]

$$\text{Gel concentration } (wt/vol\%) = \frac{Solids\ weight\ of\ gel\ (g)}{Volume\ of\ gel\ (mL)} \times 100 \qquad [1]$$

Solids weight of gel (g): Weight of precipitate (solids) obtained by centrifugation of gel (g), wherein the cyclic molecule is cyclodextrin or a derivative thereof.

7. The method according to claim 6, wherein the protein is a protein for medical use.

**Patentansprüche**

1. Gelzubereitung, die thixotrop ist und in der ein eingeschlossenes Protein vor einer physikalischen oder chemischen Beanspruchung geschützt ist, gegen Aggregation gehemmt und dadurch stabil existieren lassen wird, wobei die Gelzubereitung ein Protein, ein Verdünnungsmittel, eine oder mehrere Arten von zyklischen Molekülen und ein oder mehrere in der Gelzubereitung enthaltene Polyethylenglykole in einer Konzentration von 0,5 mg/mL bis 200 mg/mL enthält,
wobei das Protein in der Gelzubereitung in einer Konzentration von 2,5 mg/mL bis 400 mg/mL enthalten ist, und eine Gelkonzentration, dargestellt durch die folgende Formel [1], 7 % (Gew. /Vol.) bis 30 % (Gew. /Vol.) beträgt:
[Mathematische Formel 1]

$$\text{Gelkonzentration (Gew./Vol.-\%)} = \frac{Feststoffgewicht\ des\ Gels\ (g)}{Volumen\ des\ Gels\ (mL)}\ X\ 100 \quad [1]$$

Feststoffgewicht des Gels (g) : Gewicht des Niederschlags (Feststoffe) (g), der durch die Zentrifugation des Gels erhalten wurde, wobei das zyklische Molekül Cyclodextrin oder ein Derivat davon ist.

2. Gelzubereitung nach Anspruch 1, wobei die physikalische Beanspruchung mindestens eine von Wärme und Bewegung ist.

3. Gelzubereitung nach Anspruch 1, wobei die chemische Beanspruchung mindestens eine von pH-Wert, Oxidation und einer metallkatalysierten Reaktion ist.

4. Gelzubereitung nach einem der Ansprüche 1 bis 3, wobei das Cyclodextrin ausgewählt ist aus α-Cyclodextrin, β-Cyclodextrin und γ-Cyclodextrin, und das Derivat ausgewählt ist aus einem methylierten Cyclodextrin, einem maltosylierten Cyclodextrin, einem hydroxypropylierten Cyclodextrin, einem glucosylierten Cyclodextrin und einem sulfobutylierten Cyclodextrin.

5. Gelzubereitung nach einem der Ansprüche 1 bis 4, wobei das Protein ein Protein zur medizinischen Verwendung ist.

6. Verfahren zur Stabilisierung eines Proteins, umfassend das Einschließen des Proteins in eine Gelzubereitung, die eine oder mehrere Arten von zyklischen Molekülen und ein oder mehrere in der Gelzubereitung enthaltene Polyethylenglykole in einer Konzentration von 0,5 mg/mL bis 200 mg/mL enthält,
wobei das Protein in der Gelzubereitung in einer Konzentration von 2,5 mg/mL bis 400 mg/mL enthalten ist, und eine Gelkonzentration, dargestellt durch die folgende Formel[1], 7 % (Gew./Vol.) bis 30 % (Gew./Vol.) beträgt:
[Mathematische Formel 1]

$$\text{Gelkonzentration (Gew./Vol.-\%)} = \frac{Feststoffgewicht\ des\ Gels\ (g)}{Volumen\ des\ Gels\ (mL)} X\ 100 \quad [1]$$

Feststoffgewicht des Gels (g) : Gewicht des Niederschlags (Feststoffe) (g), der durch die Zentrifugation des Gels erhalten wurde, wobei das zyklische Molekül Cyclodextrin oder ein Derivat davon ist.

**7.** Verfahren nach Anspruch 6, wobei das Protein ein Protein zur medizinischen Verwendung ist.

## Revendications

**1.** Préparation de gel qui est thixotrope et dans lequel une protéine incluse est protégée contre un stress physique ou chimique, n'est plus susceptible d'agrégation, et peut ainsi être stable, où la préparation de gel contient une protéine, un diluant, un ou plusieurs type(s) de molécule(s) cyclique(s), et un ou plusieurs polyéthylène-glycol(s) contenu(s) à une concentration de 0,5 mg/mL à 200 mg/mL dans la préparation de gel,
la protéine est contenue à une concentration de 2,5 mg/mL à 400 mg/mL dans la préparation de gel, et
la concentration du gel, représentée par la formule [1] suivante, vaut de 7 % p/v à 30 % p/v :
[Formule mathématique 1]

$$\text{Concentration du gel } (\%\ p/v) = \frac{Poids\ des\ solides\ du\ gel\ (g)}{Volume\ du\ gel\ (mL)} \times 100 \qquad [1]$$

Poids des solides du gel (g) : poids du précipité (solides) obtenu par centrifugation du gel (g) ;
où la molécule cyclique est une cyclodextrine ou un dérivé de celle-ci.

**2.** Préparation de gel selon la revendication 1, dans laquelle le stress physique est au moins un parmi la chaleur et l'agitation.

**3.** Préparation de gel selon la revendication 1, dans laquelle le stress chimique est au moins un parmi le pH, l'oxydation et une réaction catalysée par un métal.

**4.** Préparation de gel selon l'une quelconque des revendications 1 à 3, dans laquelle la cyclodextrine est choisie parmi l'a-cyclodextrine, la β-cyclodextrine et la γ-cyclodextrine, et le dérivé est choisi parmi une cyclodextrine méthylée, une cyclodextrine maltosylée, une cyclodextrine hydroxypropylée, une cyclodextrine glucosylée et une cyclodextrine sulfobutylée.

**5.** Préparation de gel selon l'une quelconque des revendications 1 à 4, dans laquelle la protéine est une protéine à usage médical.

**6.** Procédé de stabilisation d'une protéine, comprenant
le fait d'inclure la protéine dans une préparation de gel qui contient un ou plusieurs type(s) de molécule(s) cyclique(s) et un ou plusieurs polyéthylèneglycol(s) contenu(s) à une concentration de 0,5 mg/mL à 200 mg/mL dans la préparation de gel,
la protéine est contenue à une concentration de 2,5 mg/mL à 400 mg/mL dans la préparation de gel, et
la concentration du gel, représentée par la formule [1] suivante vaut de 7 % p/v à 30 % p/v :
[Formule mathématique 1]

$$\text{Concentration du gel } (\% \, p/v) = \frac{\textit{Poids des solides du gel (g)}}{\textit{Volume du gel (mL)}} \times 100 \qquad [1]$$

Poids des solides du gel (g) : poids du précipité (solides) obtenu par centrifugation du gel (g) ; où la molécule cyclique est une cyclodextrine ou un dérivé de celle-ci.

7. Procédé selon la revendication 6, dans lequel la protéine est une protéine à usage médical.

FIG.1A

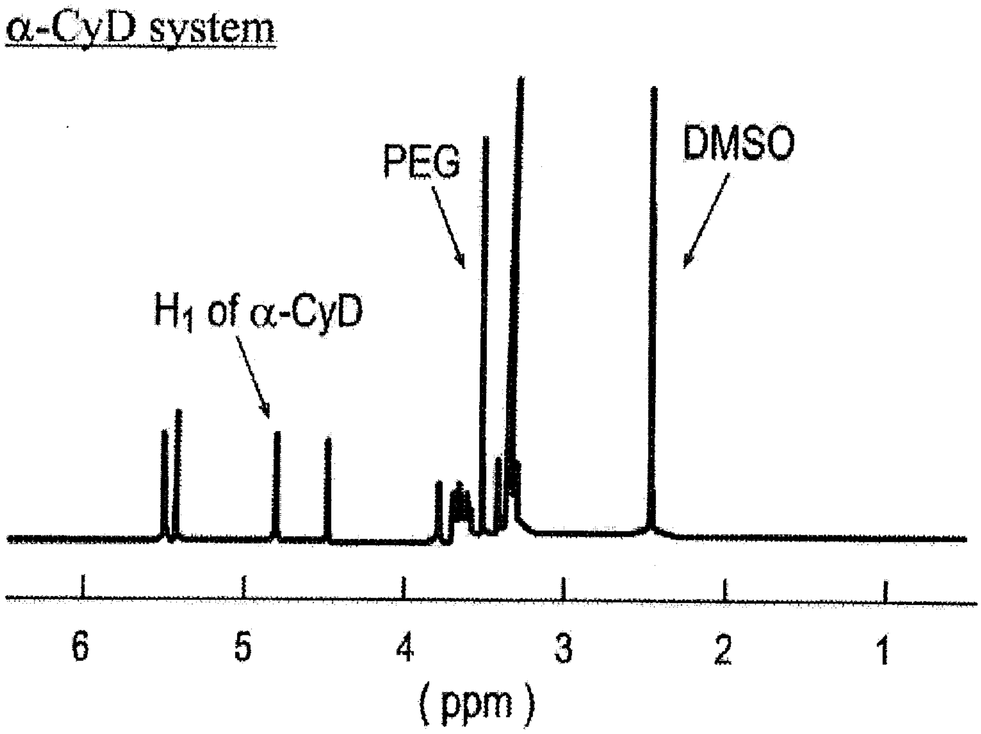

FIG.1B

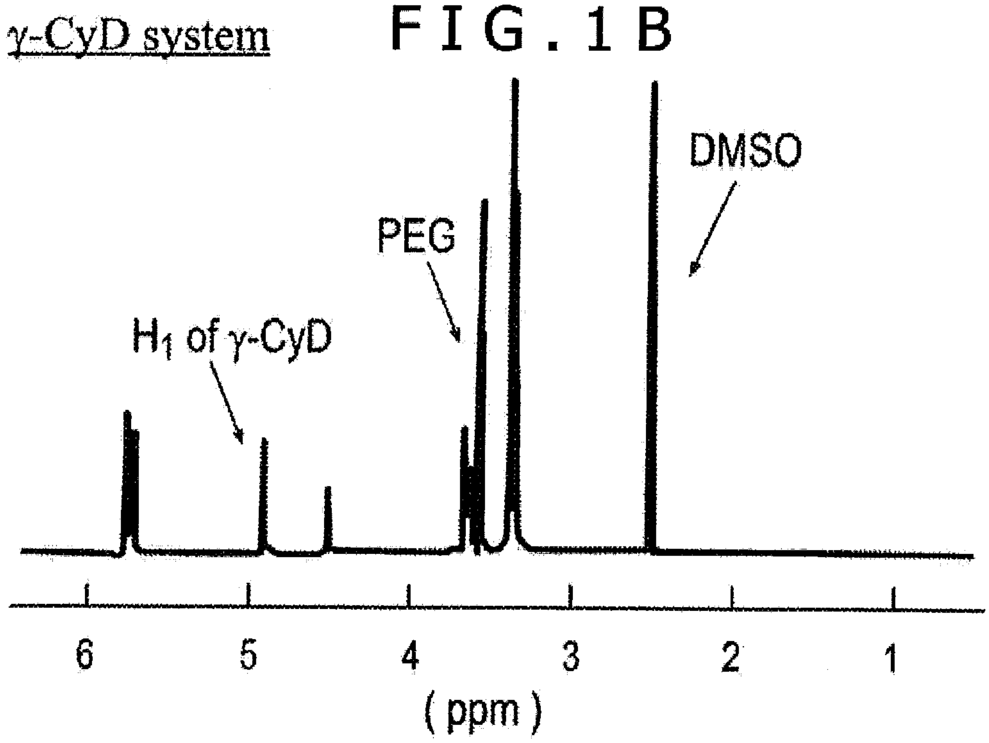
γ-CyD system
DMSO
PEG
H1 of γ-CyD
6
5
4
3
2
1
( ppm )

FIG.2A

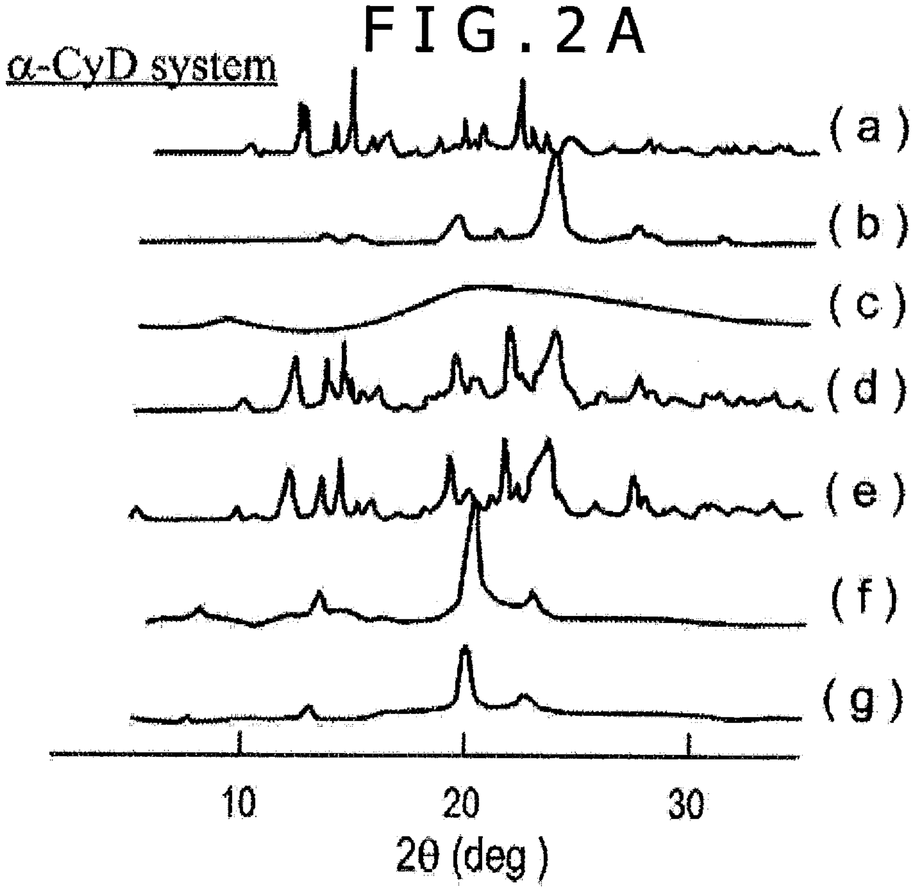
α-CyD system
( a )
( b )
( c )
( d )
( e )
( f )
( g )
10
20
30
2θ (deg )

# FIG.2B

γ-CyD system

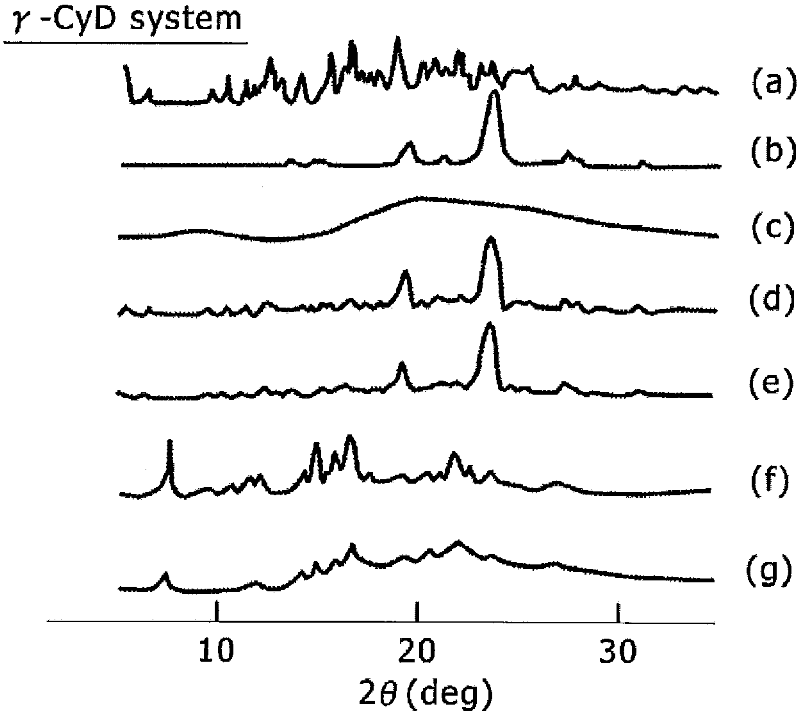


# FIG.3A

α-CyD system

HEXAGONAL COLUMNAR CHANNELS
(HEXAGONAL SYSTEM TUBULAR STRUCTURE)

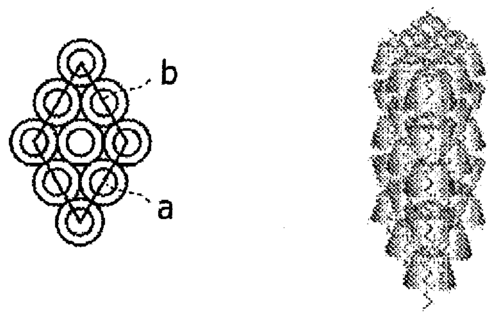


| 2θ (deg) | (hkl) | $d_{obs}$ (Å) | $d_{cal}$[1) ](Å) |
|---|---|---|---|
| 7.52 | (200) | 11.74 | 11.74 |
| 13.00 | (220) | 6.80 | 6.78 |
| 19.82 | (420) | 4.47 | 4.44 |
| 22.53 | (600) | 3.94 | 3.91 |

# FIG.3B

γ -CyD system

TETRAGONAL COLUMNAR CHANNELS
(TETRAGONAL SYSTEM TUBULAR STRUCTURE)

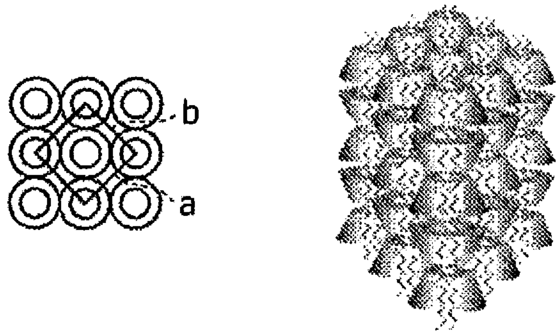


| 2θ (deg) | (hkl) | $d_{obs}$ (Å) | $d_{cal}^{2)}$ (Å) |
|---|---|---|---|
| 7.36 | (200) | 11.99 | 11.99 |
| 14.79 | (400) | 5.98 | 6.00 |
| 16.55 | (420) | 5.35 | 5.36 |
| 22.05 | (600) | 4.03 | 4.00 |

# FIG.4A

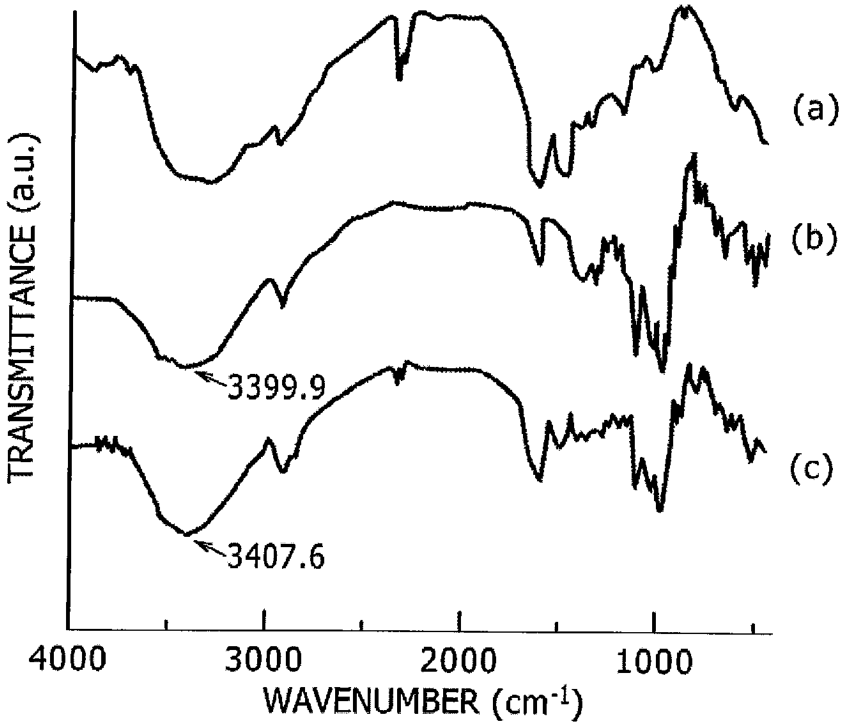

FIG.4B

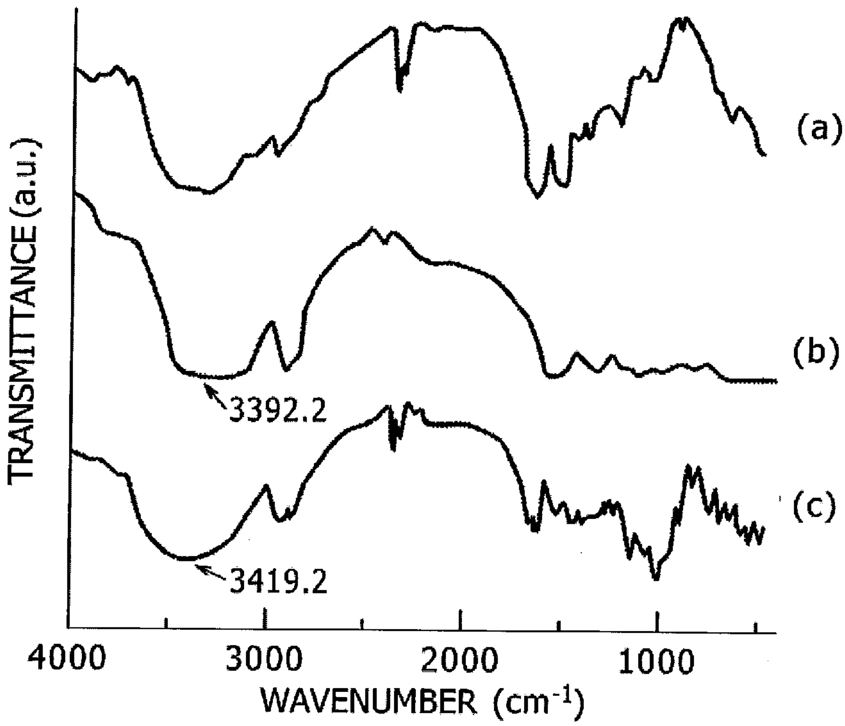
TRANSMITTANCE (a.u.)
(a)
(b)
3392.2
(c)
3419.2
4000
3000
2000
1000
WAVENUMBER (cm-1)

FIG.5A

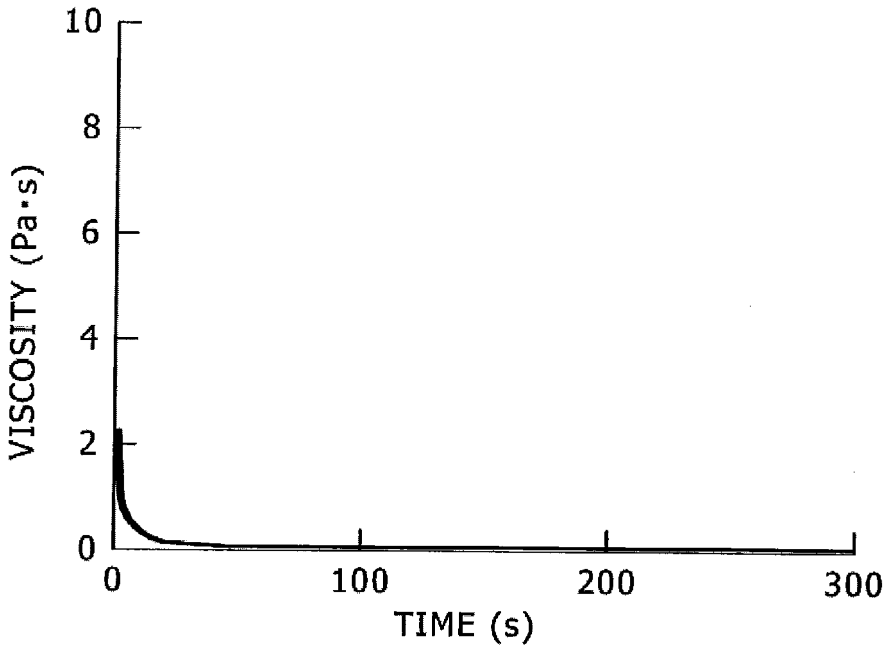
10
8
6
4
2
0
VISCOSITY (Pa·s)
0
100
200
300
TIME (s)

# FIG.5B

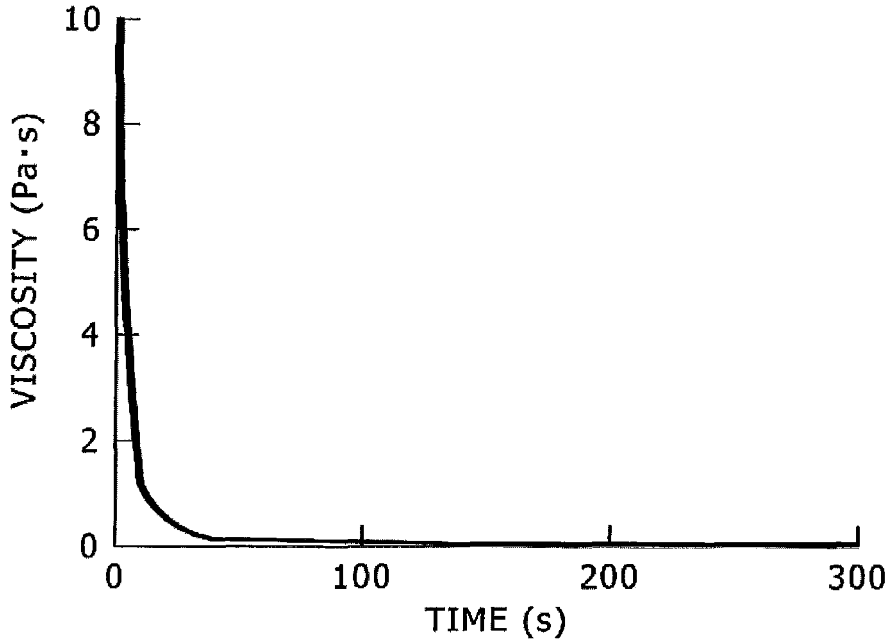
10
8
6
4
2
0
VISCOSITY (Pa·s)
0
100
200
300
TIME (s)

# FIG.6A

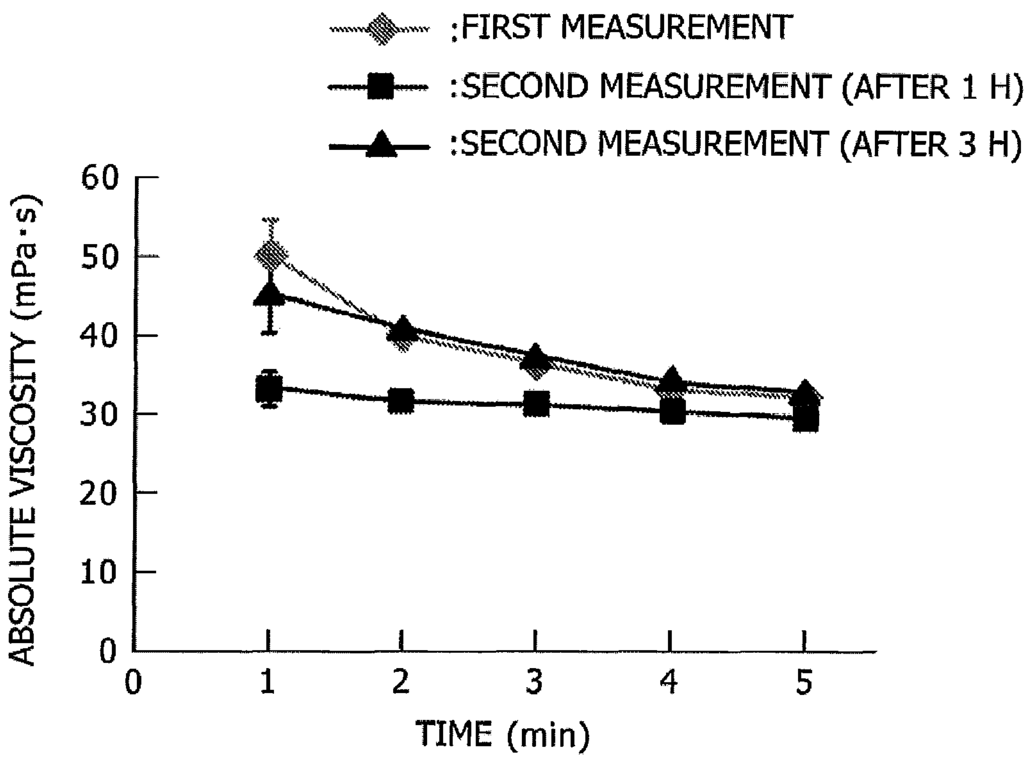
:FIRST MEASUREMENT
:SECOND MEASUREMENT (AFTER 1 H)
:SECOND MEASUREMENT (AFTER 3 H)
60
50
40
30
20
10
0
ABSOLUTE VISCOSITY (mPa·s)
0
1
2
3
4
5
TIME (min)

FIG.6B

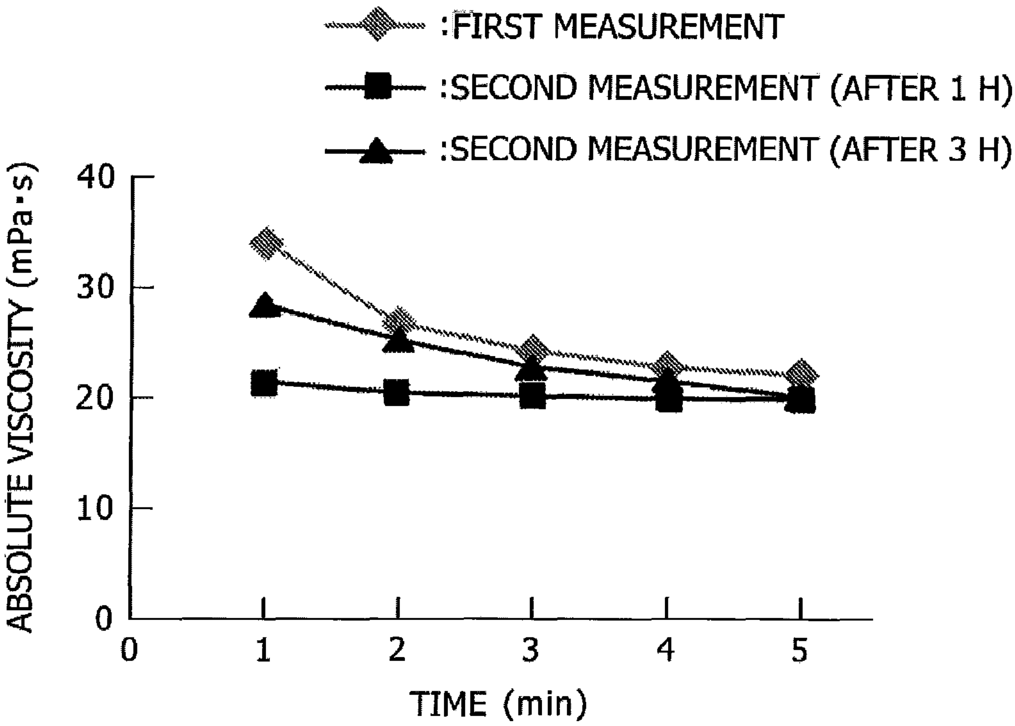
:FIRST MEASUREMENT
:SECOND MEASUREMENT (AFTER 1 H)
:SECOND MEASUREMENT (AFTER 3 H)
ABSOLUTE VISCOSITY (mPa·s)
40
30
20
10
0
0
1
2
3
4
5
TIME (min)

FIG.7

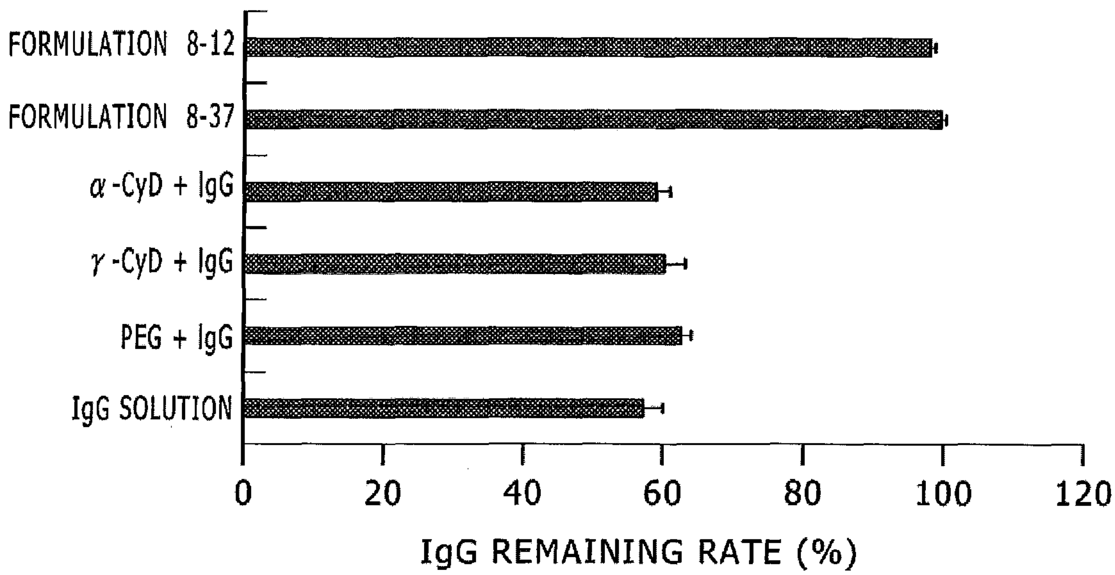
FORMULATION 8-12
FORMULATION 8-37
α-CyD + IgG
γ-CyD + IgG
PEG + IgG
IgG SOLUTION
0
20
40
60
80
100
120
IgG REMAINING RATE (%)

FIG. 8

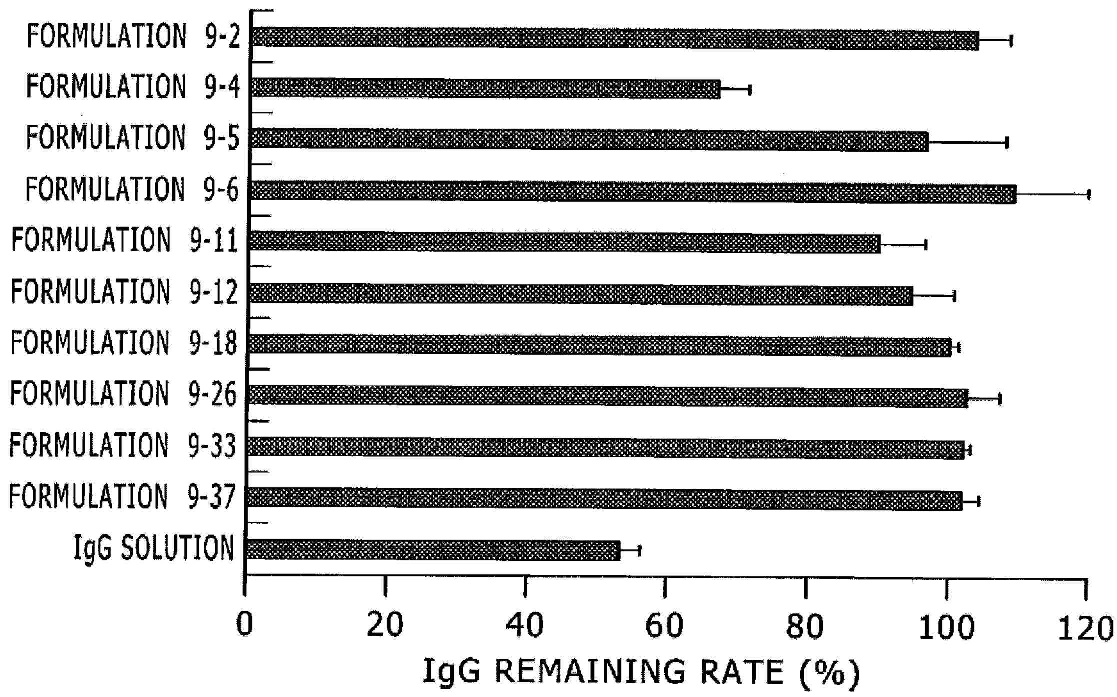
FORMULATION 9-2
FORMULATION 9-4
FORMULATION 9-5
FORMULATION 9-6
FORMULATION 9-11
FORMULATION 9-12
FORMULATION 9-18
FORMULATION 9-26
FORMULATION 9-33
FORMULATION 9-37
IgG SOLUTION
0
20
40
60
80
100
120
IgG REMAINING RATE (%)

FIG. 9

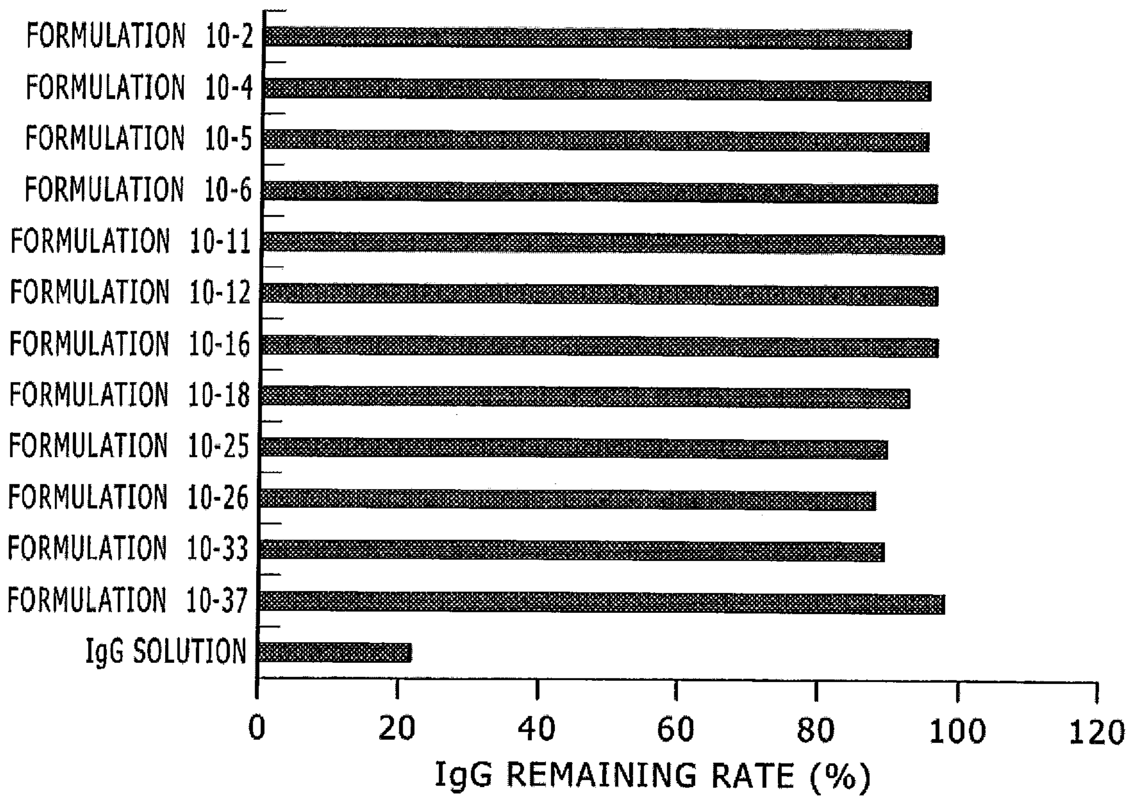
FORMULATION 10-2
FORMULATION 10-4
FORMULATION 10-5
FORMULATION 10-6
FORMULATION 10-11
FORMULATION 10-12
FORMULATION 10-16
FORMULATION 10-18
FORMULATION 10-25
FORMULATION 10-26
FORMULATION 10-33
FORMULATION 10-37
IgG SOLUTION
0
20
40
60
80
100
120
IgG REMAINING RATE (%)

FIG.10

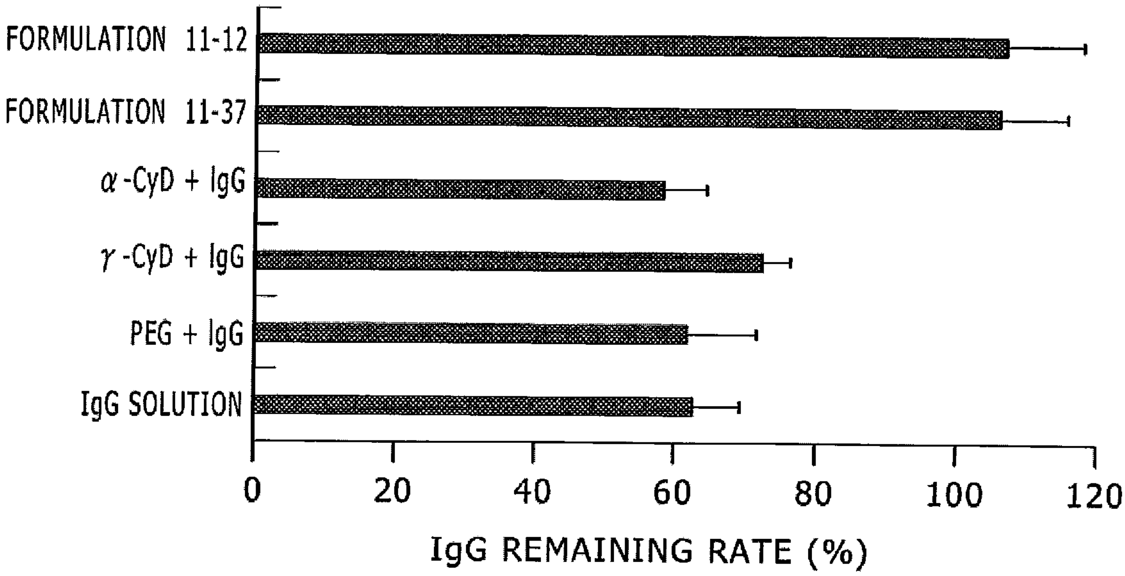
FORMULATION 11-12
FORMULATION 11-37
α-CyD + IgG
γ-CyD + IgG
PEG + IgG
IgG SOLUTION
0
20
40
60
80
100
120
IgG REMAINING RATE (%)

FIG.11

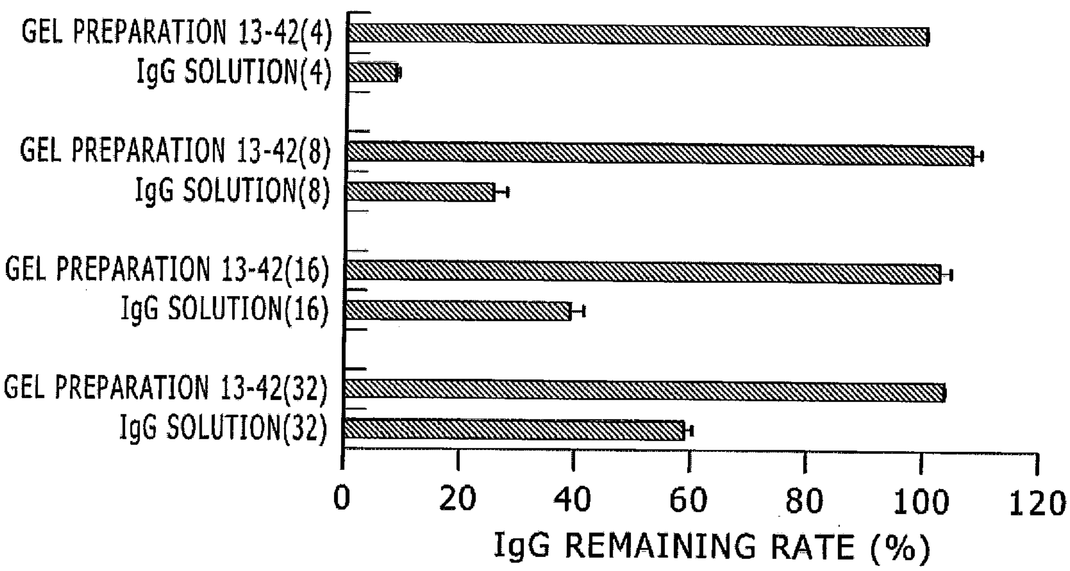
GEL PREPARATION 13-42(4)
IgG SOLUTION(4)
GEL PREPARATION 13-42(8)
IgG SOLUTION(8)
GEL PREPARATION 13-42(16)
IgG SOLUTION(16)
GEL PREPARATION 13-42(32)
IgG SOLUTION(32)
0
20
40
60
80
100
120
IgG REMAINING RATE (%)

FIG.12

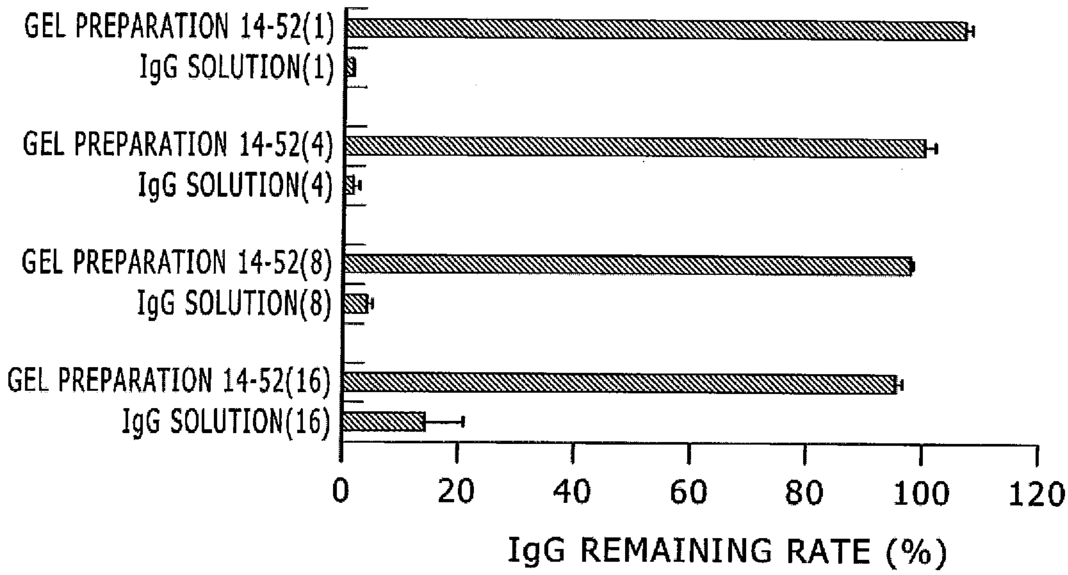


FIG.13A

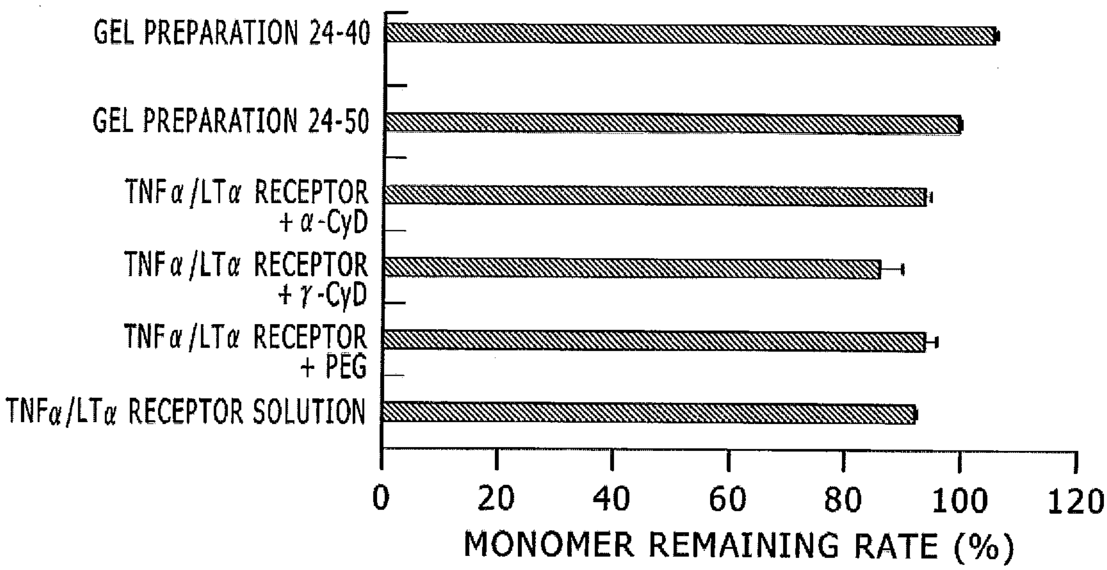

FIG.13B

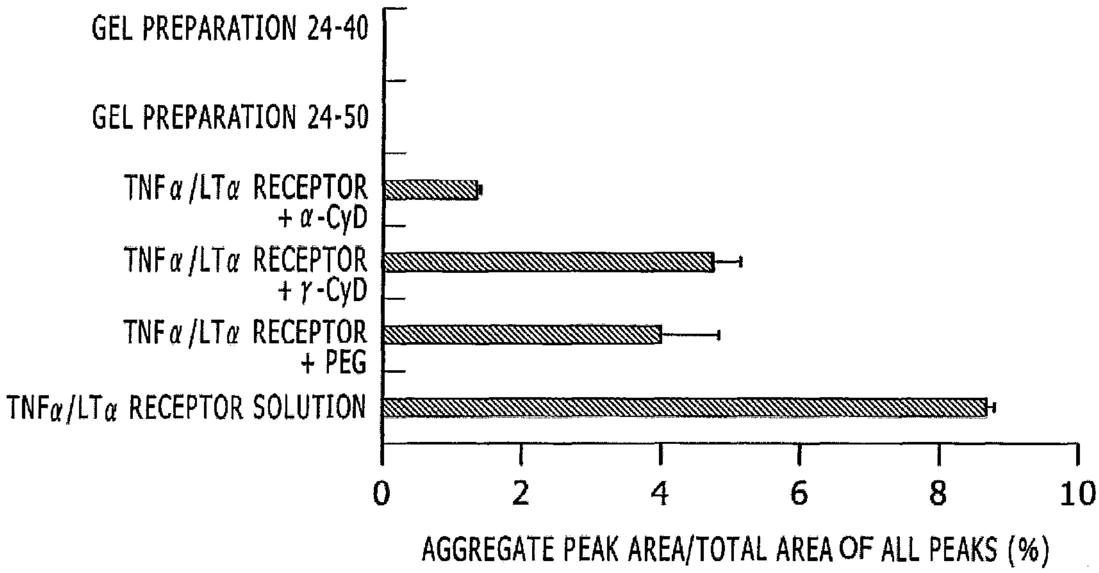


FIG.14

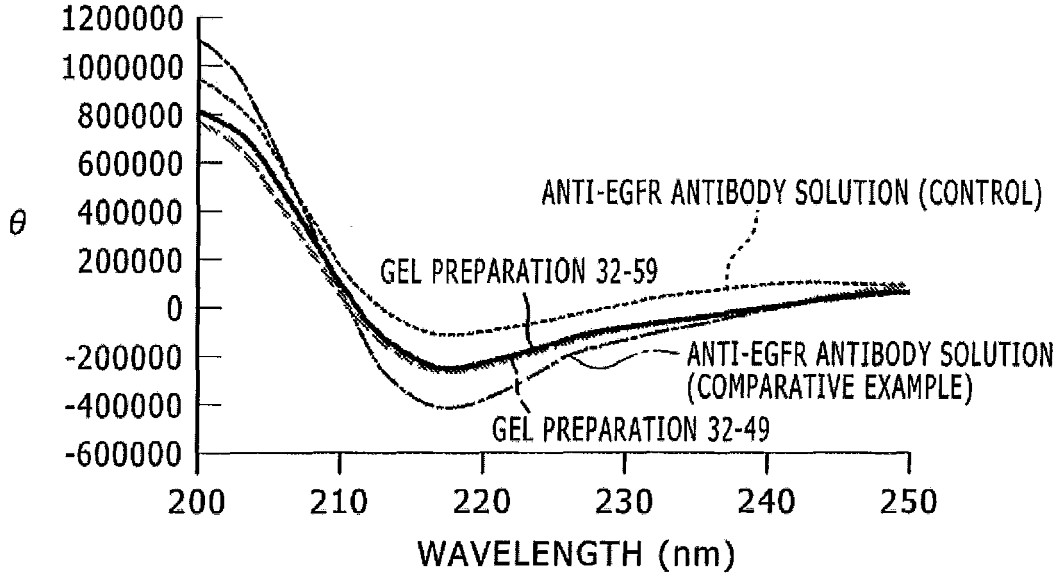

FIG.15A

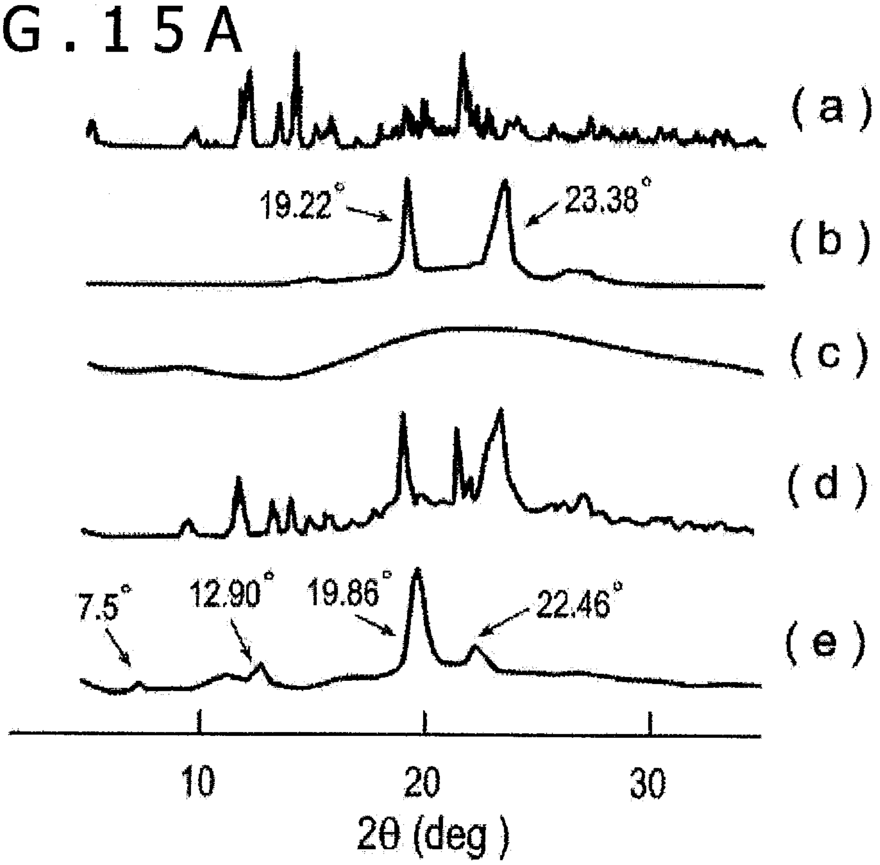


FIG.15B

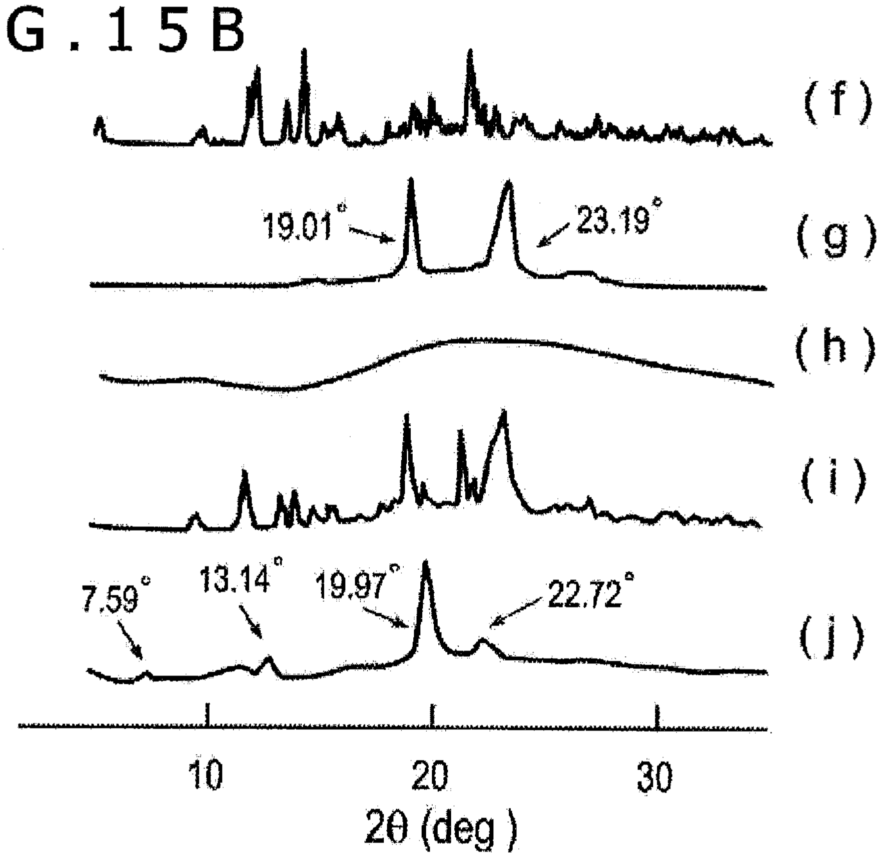

FIG.15C

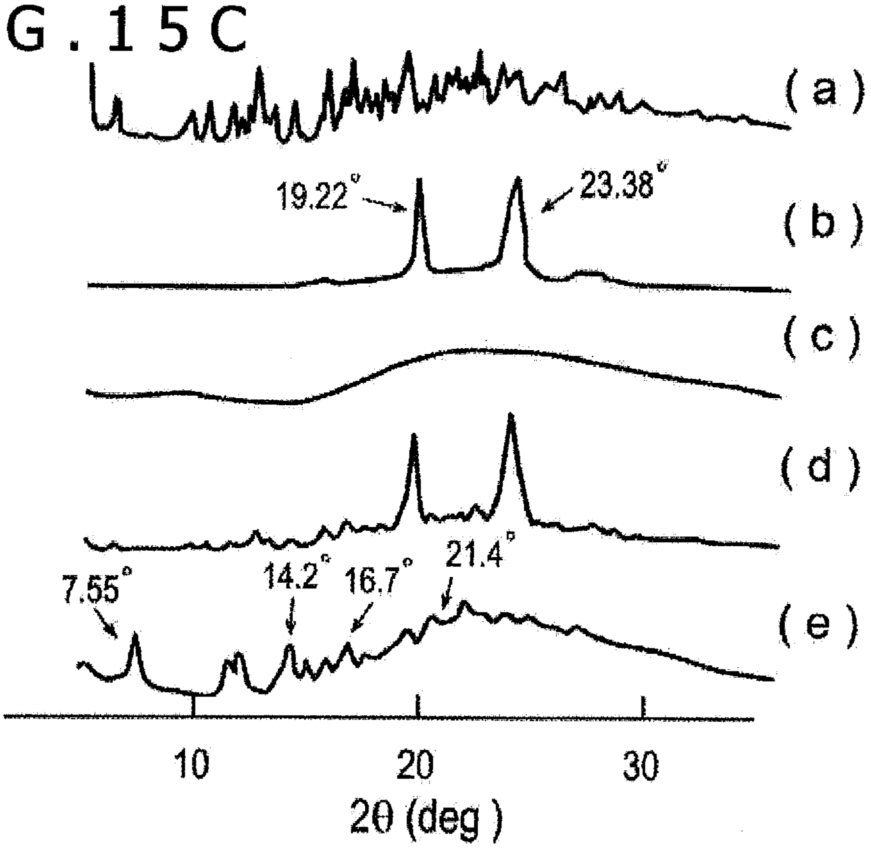


FIG.15D

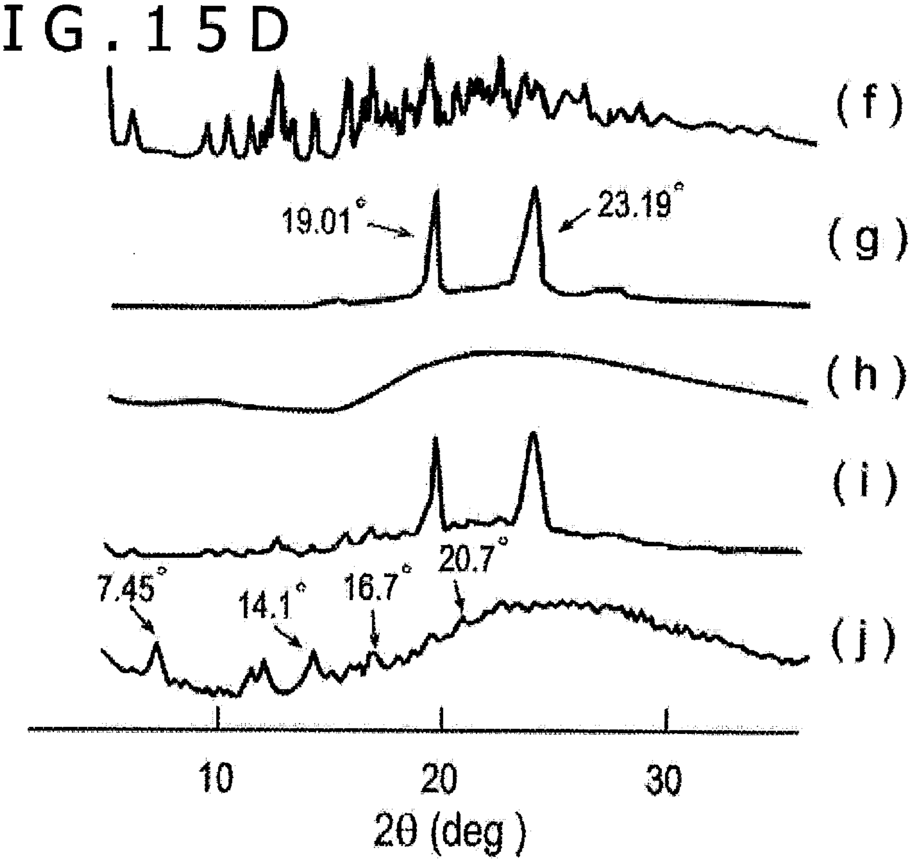

FIG.16

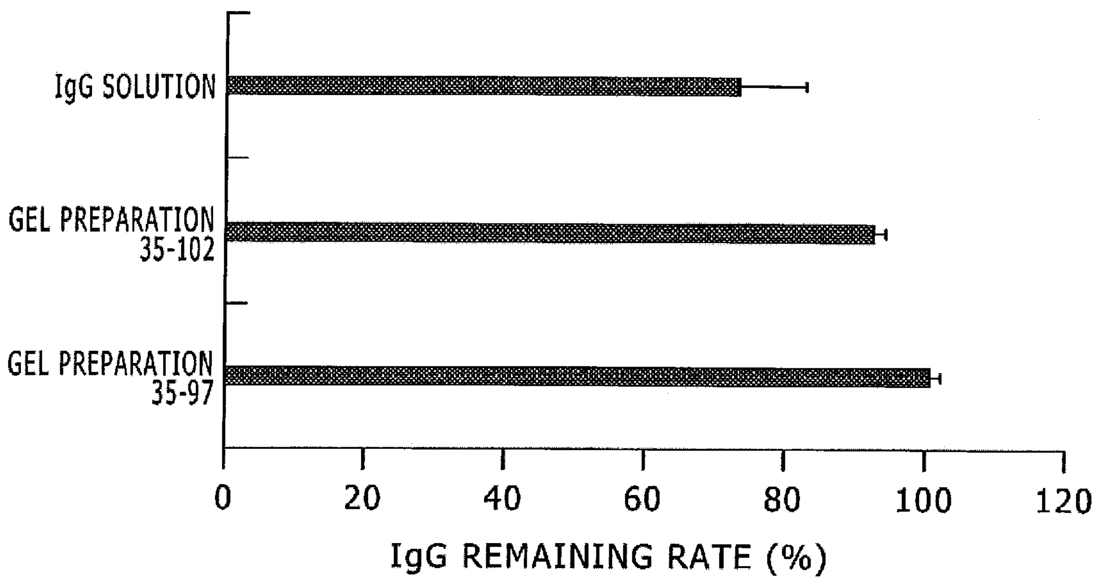
IgG SOLUTION
GEL PREPARATION 35-102
GEL PREPARATION 35-97
0
20
40
60
80
100
120
IgG REMAINING RATE (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4783441 A
- WO 2002013859 A
- US 7648702 B
- JP 4219932 B
- US 8496930 B
- WO 2008076819 A
- WO 9955310 A
- US 6541606 B
- US 2002019369 A
- US 2007248674 A
- WO 9955310 PCT


### Non-patent literature cited in the description


- **SCHELLEKENS, H.** *Clin. Therapeutics,* 2002, vol. 24 (11), 1720-1740